(19) Europäisches Patentamt / European Patent Office / Office européen des brevets

(11) **EP 1 948 128 B1**

(12) **FASCICULE DE BREVET EUROPEEN**

(45) Date de publication et mention de la délivrance du brevet:
**15.03.2017   Bulletin 2017/11**

(21) Numéro de dépôt: **06831309.7**

(22) Date de dépôt: **26.10.2006**

(51) Int Cl.:
*A61Q 19/08* (2006.01)       *A61K 36/77* (2006.01)
*A61K 8/97* (2017.01)        *G01N 33/68* (2006.01)
*G01N 33/573* (2006.01)      *C12Q 1/68* (2006.01)
*A61P 17/06* (2006.01)       *A61P 17/00* (2006.01)
*A61P 35/00* (2006.01)       *A61P 37/06* (2006.01)
*A61K 36/48* (2006.01)       *A61K 36/17* (2006.01)
*A61K 36/185* (2006.01)      *A61K 36/54* (2006.01)

(86) Numéro de dépôt international:
**PCT/FR2006/051117**

(87) Numéro de publication internationale:
**WO 2007/048985 (03.05.2007 Gazette 2007/18)**

(54) **SUBSTANCE POUR RESTAURER UNE CO-EXPRESSION ET UNE INTERACTION NORMALES ENTRE LES PROTEINES LOX ET NRAGE**

SUBSTANZ ZUR WIEDERHERSTELLUNG DER NORMALEN CO-EXPRESSION UND INTERAKTION ZWISCHEN LOX-UND NRAGE-PROTEINEN

SUBSTANCE FOR RESTORING NORMAL CO-EXPRESSION AND INTERACTION BETWEEN LOX AND NRAGE PROTEINS

(84) Etats contractants désignés:
**AT BE BG CH CY CZ DE DK EE ES FI FR GB GR HU IE IS IT LI LT LU LV MC NL PL PT RO SE SI SK TR**

(30) Priorité:  **28.10.2005   FR 0511112**

(43) Date de publication de la demande:
**30.07.2008   Bulletin 2008/31**

(60) Demande divisionnaire:
**10186248.0 / 2 345 405**

(73) Titulaires:
• **BASF Beauty Care Solutions France SAS**
  **69007 Lyon (FR)**
• **Centre National de la Recherche Scientifique**
  **75016 Paris (FR)**
• **Université Claude Bernard Lyon I**
  **69625 Villeurbanne Cedex (FR)**

(72) Inventeurs:
• **BOUEZ, Charbel**
  **75007 Paris (FR)**
• **GLEYZAL, Claudine**
  **01350 Culoz (FR)**
• **ORLY, Isabelle**
  **F-69540 Irigny (FR)**
• **ANDRE, Valérie**
  **F-69420 Ampuis (FR)**
• **SOMMER, Pascal**
  **F-69230 Saint Genis Laval (FR)**
• **REYMERMIER, Corinne**
  **F-69390 Charly (FR)**
• **DAMOUR, Odile**
  **F-69230 Saint Genis Laval (FR)**
• **PERRIER, Eric**
  **F-38138 Les Cotes d'Areyl (FR)**

(74) Mandataire: **Chantraine, Sylvie Hélène et al**
**Cabinet Beau de Loménie**
**158, rue de l'Université**
**75340 Paris Cedex 07 (FR)**

(56) Documents cités:
| EP-A- 1 059 086 | EP-A- 1 570 851 |
| WO-A-01/19850 | WO-A-01/20335 |
| WO-A-94/23732 | WO-A-02/076487 |
| WO-A-03/084522 | WO-A-2004/064801 |
| WO-A-2005/092121 | WO-A-2006/029484 |
| DE-A1- 19 853 425 | FR-A- 2 849 992 |
| FR-A- 2 855 969 | FR-A1- 2 723 096 |
| FR-A1- 2 777 192 | FR-A1- 2 779 348 |

**FR-A1- 2 837 702**    **US-A1- 2004 043 083**
**US-A1- 2004 071 791**    **US-A1- 2004 185 122**
**US-A1- 2005 025 846**    **US-A1- 2005 031 572**
**US-A1- 2005 181 080**    **US-A1- 2005 191 375**

- **DATABASE WPI Week 200649 Derwent Publications Ltd., London, GB; AN 2006-472863 XP002425451 & CN 1 723 951 A (PHARM RES INST SHANDONG PROV MEDICAL SCI ACAD) 25 janvier 2006 (2006-01-25)**

Remarques:
Le dossier contient des informations techniques présentées postérieurement au dépôt de la demande et ne figurant pas dans le présent fascicule.

## Description

L'invention concerne l'utilisation d'une substance pour restaurer une co-expression et une interaction normales entre les protéines LOX et NRAGE.

## ETAT DE L'ART

### 1) Généralités

Dans les organismes multicellulaires, l'homéostasie est maintenue par un équilibre entre la prolifération cellulaire, la différenciation des cellules envers une fonction définie et la mort des cellules, programmée ou non.

La peau, par exemple, peut être considérée comme un organe dont une des fonctions spécifiques est de protéger l'organisme des agressions extérieures. L'étanchéité envers l'extérieur est assurée par une couche cornée hautement kératinisée composée de cellules mortes. La vie des cellules de l'épiderme, les kératinocytes, est donc rythmée par une succession d'évolutions successives conduisant à cette différenciation en cellules kératinisées et mortes: (1) maintenance de cellules initiatrices (cellules « souches » de la couche basale de l'épiderme), (2) division asymétrique (chaque cellule souche donnant deux cellules soeurs), (3) différenciation (formation des couches suprabasales de l'épiderme à partir d'une des cellules « soeurs initiatrices »), (4) acquisition d'une résistance à la mort cellulaire de type apoptotique, (5) évolution vers des cellules cornifiées et (6) mort cellulaire programmée des couches cellulaires supérieures de l'épiderme. Toute modification de cet équilibre peut être pathologique.

Plusieurs molécules et mécanismes biologiques sont connus de l'homme de l'art, qui rendent compte de certains aspects des phénomènes cellulaires de prolifération, de différenciation et d'apoptose. Toutefois, aucune donnée ne renseigne réellement sur un quelconque mécanisme qui lierait ces trois phénomènes, et qui constituerait ainsi une cible sur laquelle agir en vue de les contrôler et de les réguler.

### 2) LOX (protéine)

LOX appartient à la famille des lysyl oxydases (LO) qui sont des amine oxydases dépendantes du cuivre. À ce jour, 5 gènes *LO* ont été caractérisés : *LOX*, *LOXL*, *LOXL2*, *LOXL3* et *LOXL4.* Si LOX est connue pour son rôle dans à la réticulation du collagène *ex vivo et in vivo*, LOXL est clairement associée à l'homéostasie des fibres élastiques. Le rôle *in vivo* des autres isoformes n'est pas connu.

Les LOs sont synthétisées par des cellules diverses telles que les fibroblastes, les cellules musculaires lisses, les cellules endothéliales, et les kératinocytes. Cette enzyme est donc présente dans de nombreux tissus tels que la peau, le foie, les reins, la rate et l'aorte, aussi bien au niveau extracellulaire qu'intracellulaire.

### 2.1) Stabilisation de la matrice extracellulaire - rôle extracellulaire

Le rôle extracellulaire de LOX, désormais bien connu, consiste à stabiliser la matrice extracellulaire (MEC) des tissus conjonctifs. LOX a en effet pour fonction de réticuler les collagènes fibrillaires et l'élastine. Pour cela, elle catalyse la désamination oxydative de résidus lysyls et hydroxylysyls des molécules de pro-collagène fibrillaires et de la tropoélastine, réaction qui s'accompagne de la libération d'ammoniaque et de peroxyde d'hydrogène. Les résidus aldéhydes formés se condensent alors spontanément avec des fonctions aldéhydes ou amines voisines, conduisant à des liaisons intra et intermoléculaires. Ces condensations sont à l'origine des pontages que l'on trouve dans les fibres de collagènes et d'élastine. LOX, et les LOs, sont donc étudiées dans les domaines biomédicaux qui impliquent une évolution de la MEC (vieillissement, fibroses, cancer, cicatrisation, pathologies ostéoarticulaires et cardiovasculaires, angiogenèse).

Il a été démontré *in vitro* que les nitriles organiques étaient des inhibiteurs irréversibles de LOX. Ainsi, le ß-aminoproprionitrile (ß-APN) se fixe, de manière compétitive avec les substrats alkyl amine, sur le site actif de LOX ; les LOs utilisent le ß -APN comme substrat, formant ainsi une base de Schiff mais sans libération du produit aldéhyde, ce qui bloque le site actif de manière covalente sans avoir d'effet sur la synthèse. Sur cette base, les inventeurs ont décrit l'utilisation de différents inhibiteurs des lysyl oxydases, dont le ß-APN, pour éviter la dédifférenciation de certains types cellulaires (chondrocytes...) qui se produit systématiquement lorsque ces cellules sont cultivées (Farjanel et al, brevet français 01.10443, CNRS, Utilisation d'inhibiteurs des lysyl oxydases pour la culture cellulaire et le génie tissulaire n° déposée le 3 août 2001, n° publication : 2 828 206). Toutefois, ce document concerne les lysyl oxydases dans leur ensemble et pas particulièrement l'isoforme LOX ; de plus, il porte exclusivement sur l'inhibition de la dédifférenciation des cellules en culture *in vitro,* ne donnant aucun enseignement sur le rôle des lysyl oxydases, ni a *fortiori* de LOX, dans le maintien de l'équilibre entre prolifération, différenciation et apoptose, pas plus qu'il ne fournit d'enseignement sur de nouveaux partenaires ou substrats des lysyl oxydases, ni de LOX.

### 2.2) Rôle intracellulaire

Le rôle intracellulaire de LOX, et des LOs en général, est moins connu. Ainsi, bien qu'il soit admis que LOX participe à la régulation du développement, à la différenciation, à la mobilité ou à la sénescence cellulaire (Csizar, Lysyl oxydases : A novel multifunctional amine oxydase familty, Nucleic Acid research and Molecular Biology, 2001, 70 : 2-28), les mécanismes moléculaires sous-jacents ne sont pas élucidés.

### 2.2.1) LOX et homéostasie cellulaire

L'implication possible de LOX dans la régulation de l'homéostasie cellulaire (maintien d'un équilibre physiologique entre prolifération, différenciation et apoptose) est couramment acceptée (Jeay et al., Lysyl oxydase inhibits Ras-mediated transformation by preventing activation of NF-KB, Mol. Cell. Biol., 2003, 23 : 2251-2263), toutefois les mécanismes sous-jacents sont encore inconnus de l'homme de l'art.
Ainsi l'homme de l'art dispose aujourd'hui de quelques données indiquant une relation possible entre LOX et différenciation d'une part, et LOX et prolifération/transformation cellulaire, d'autre part. Cependant, l'art antérieur ne fait pas état d'une relation possible entre LOX et apoptose.

### 2.2.2) LOX et différenciation épidermique

LOX a été localisée dans l'épiderme, avec une expression régulée en fonction du niveau de différenciation des kératinocytes (Noblesse et al, Lysyl oxydase like and lysyl oxydase are present in the dermis and epidermis of a skin equivalent and in human skin and are associated to elastic fibers, J. Invest. Dermatol., 2004, 122 621-630). Toutefois, les études réalisées à ce jour n'ont pas permis de définir le rôle de LOX dans les kératinocytes, ni de rechercher ses partenaires dans ces cellules, qui ne font pas (ou peu) de collagène et d'élastine.

### 2.2.3) LOX et transformation cellulaire

Le gène *LOX* est clairement associé au maintien du phénotype non tumoral des cellules.
Deux hypothèses ont été émises pour expliquer ce rôle de LOX. La première hypothèse suggère que la réticulation de la MEC induit un environnement tridimensionnel favorable au maintien de l'état non tumoral. Cette hypothèse est étayée par le fait que LOX et LOXL ne sont plus exprimées lorsque les cancers deviennent invasifs, alors qu'elles sont présentes dans les cancers *in situ* (Peyrol et al., Lysyl oxydase gene expression in the stromal reaction to in situ and invasive ductal breast carcinoma., Am J Pathol. Feb; 150(2):497-507 1997). L'autre hypothèse concerne le rôle intracellulaire des LOs, sur des substrats qu'il reste à découvrir à l'homme de l'art (Li et al. Localization and activity of lysyl oxydase within nuclei of fibrogenic cells., Proc Natl Acad Sci USA., 1997 Nov 25;94(24):12817-22.).
Il s'avère en effet que l'enzyme LOX est un suppresseur de l'oncogène *ras*, et que des mutations somatiques dans le gène sont associées à divers cancers (Contente et al., Expression of gene rrg is associated with reversion of NIH 3T3 transformed by LTR-c-H-ras, Science, 1990, 249 : 796-798; Csiszar et al., Somatic mutations of the lysyl oxidase gene on chromosome 5q23.1 in colorectal tumors, Int. J. Cancer, 2002, 97 : 636-642). De récents travaux montrent que le faible niveau d'expression de LOX dans les fibroblastes transformés par *ras* est dû à l'activité du facteur de croissance autocrine FGF-2, et que la drogue anti-tumorale suramine permet la ré-induction de l'expression de l'enzyme (Palamakumbura et al., Autocrine growth factor regulation of lysyl oxydase expression in transformed fibroblasts., J Biol Chem., 2003, Aug 15;278(33):30781-7 ; Epub 2003 Jun 4). D'autre part, il a été montré que la ré-expression de LOX dans ces fibroblastes inhibe leur croissance en agar mou, et ce, en agissant sur la voie de signalisation NF-kB à travers la régulation de la localisation de la protéine AKT (Jeay et al., Lysyl oxydase inhibits Ras-mediated transformation by preventing activation of NF-B, Mol. Cell. Biol., 23 : 2251-2263, 2003).
Comme dans le cas d'autres suppresseurs de tumeur, LOX agit probablement selon la disponibilité et la régulation de ses substrats et partenaires cellulaires, mais ces derniers restent inconnus de l'homme de l'art.

### 2.2.4) Apoptose

Le terme d'apoptose est utilisé pour décrire une forme particulière de mort cellulaire, présentant des caractéristiques morphologiques différentes de celles de la nécrose.
L'apoptose est un processus de mort cellulaire programmée qui requiert l'acquisition de caspases. Au cours de ce processus, les cellules acquièrent des caractéristiques morphologiques particulièrement remarquables, comme la condensation de la chromatine et la fragmentation du noyau, conduisant à leur autodestruction et à leur élimination du tissu sans endommager les cellules voisines.
L'apoptose correspond à la mort naturelle des cellules, au cours de leur développement, ou pendant l'homéostasie.

L'art antérieur renseigne sur un certain nombre d'acteurs et de mécanismes qui, à travers les processus cellulaires d'apoptose et de différenciation, sont impliqués dans la régulation du cycle cellulaire.

**3) NRAGE (protéine)**

Ainsi, il est connu, notamment, une famille de protéines qui sont impliquées dans la régulation du cycle cellulaire, de la différenciation et de l'apoptose. Il s'agit de la famille des protéines MAGE (Melanoma Associated Antigen), dont l'un des membres, la protéine NRAGE, également appelée « neurotrophin-receptor-interacting MAGE homolog », est particulièrement connu pour son rôle pro-apoptotique *via* le facteur neurotrophique NGF.

La protéine NRAGE compte 778 acides aminés. Elle porte un premier domaine caractéristique des protéines MAGE: le MAGE Homology Domain (MHD-1) en région centrale et, en région N-terminale, un second domaine : MHD-2, uniquement présent chez certaines isoformes. Ces deux domaines présentent des zones riches en résidus lysyls. Entre ces deux domaines, il existe une région appelée IRD (Interpersed Repeat Domain) qui n'existe dans aucune autre protéine connue à ce jour.

La protéine NRAGE est impliquée dans le contrôle de l'apoptose à travers différentes voies.

- Par son interaction avec p75NTR, qui est le récepteur "à basse affinité" des facteurs neutrophiles (NGF) ou du TNF, NRAGE peut bloquer la progression du cycle cellulaire et est ainsi pro-apoptotique à travers la voie des caspases.
- NRAGE est aussi pro-apoptotique en interagissant avec les inhibiteurs cytoplasmiques de protéines apoptotiques IAP.
- NRAGE peut également agir directement sur l'activité de d'homéo-facteurs nucléaires, comme les facteurs Msx et Dlx qui interviennent dans la régulation morphogène des tissus.

Bien que l'expression de NRAGE soit ubiquitaire, l'art antérieur ne fait pas état de sa présence au niveau de la peau.

L'art antérieur n'apporte non plus aucun élément qui renseignerait sur une relation éventuelle entre LOX et apoptose, pas plus que sur un lien entre LOX et NRAGE.

**4) Conclusion sur l'art antérieur**

L'art antérieur ne fournit pas l'identité de nouveaux partenaires ou substrats de LOX, notamment intervenant dans le maintien de l'équilibre cellulaire entre prolifération, différenciation, et apoptose, l'art antérieur ne fournissant d'ailleurs l'identité de ces nouveaux partenaires ou substrats ni dans les cellules épithéliales (en particulier les kératinocytes), ni dans quelque autre type cellulaire que ce soit.

L'art antérieur ne renseigne pas sur les éventuelles variations d'expression de LOX au niveau épidermique (notamment exprimée par les kératinocytes), en fonction de l'age, de l'existence ou non d'exposition aux UV ou à d'autres types d'agressions, ou en cas de pathologies affectant la peau (psoriasis, réponse de l'hôte contre une greffe, cancers...).

L'art antérieur ne décrit pas l'implication de LOX dans l'apoptose.

L'art antérieur ne renseigne pas sur l'éventuelle présence de NRAGE dans la peau, qu'il s'agisse de peau saine, de peau altérée par l'age ou par les UV, ou ayant subi d'autres types d'agressions, ou de peau pathologique.

L'art antérieur ne dispose de modèles connus pour étudier NRAGE dans des cellules d'origine épithéliale, ni dans l'épiderme.

L'art antérieur ne renseigne pas sur une quelconque interaction de LOX avec NRAGE, et ceci quel que soit le tissu.

L'art antérieur ne fournit pas de modèle permettant l'identification de principes actifs capables de moduler l'expression de LOX et/ou de NRAGE dans des kératinocytes.

D'autre part, à ce jour l'expérimentation animale est interdite en Europe pour certaines applications et l'expérimentation humaine est éthiquement discutée. Il n'est donc pas acceptable pour les inventeurs d'effectuer une méthode de criblage mettant en oeuvre des animaux ou des êtres humains.

Dans le modèle tridimensionnel MIMESKIN® (Coletica, France), LOX est exprimée au niveau de l'épiderme, avec une expression régulée en fonction du niveau de différenciation des kératinocytes (Noblesse et al, 2004). Au cours de ces études, n'ont été recherchées ni l'expression de NRAGE, ni l'existence d'une éventuelle relation entre LOX et l'apoptose. Ainsi, il n'était pas évident pour l'homme du métier de s'intéresser à la modulation de l'expression de LOX et/ou de NRAGE pour réguler l'homéostasie cellulaire reposant sur l'équilibre entre prolifération, différenciation et apoptose, dans les cas où elle est perturbée (age, stress, pathologies), qui constitue dès lors un problème technique nouveau.

L'art antérieur ne permet donc pas la fourniture de principes actifs capables de moduler l'expression de partenaires intracellulaires de LOX (comme NRAGE) associée ou non à une modulation de l'expression de LOX dans le but d'agir sur la régulation cellulaire. Dans ce cadre, il est également très difficile d'obtenir des critères objectifs permettant de juger de l'impact de ces actifs.

## BUTS DE L'INVENTION

L'invention a principalement pour but de résoudre les problèmes techniques énoncés ci-dessus et notamment vise à réguler l'équilibre cellulaire entre prolifération, différenciation et apoptose, dans les cas où il est perturbé dans le cas de peau altérée par l'age.

L'invention concerne ainsi l'utilisation selon la revendication 1. En particulier le principe actif stimulant l'expression de LOX permet de réguler l'équilibre cellulaire entre prolifération, différenciation et apoptose dans les cas où il est perturbé dans le cas de peau altérée par l'age.

## DESCRIPTION DE L'INVENTION

Dans l'ensemble de la description, les inventeurs entendent par les termes :

« LOX », l'isoforme de la protéine humaine de la lysyl oxydase LOX, en particulier définie par la séquence d'acides aminés SEQ ID n°1 ;

« NRAGE », la protéine humaine NRAGE, en particulier définie par la séquence d'acides aminés SEQ ID n°2 ;

« moduler l'expression de LOX », la modulation du gène codant LOX, et notamment la modulation de l'expression de l'ARN messager codant LOX, mais aussi la modulation de la synthèse de LOX à partir de cet ARN messager, ainsi que la modulation de l'activité de LOX ;

« moduler l'expression de NRAGE », la modulation du gène codant NRAGE, et notamment la modulation de l'expression de l'ARN messager codant NRAGE, mais aussi la modulation de la synthèse de NRAGE à partir de cet ARN messager, ainsi que la modulation de l'effet biologique de NRAGE.

Ces modulations doivent permettre de ré-induire un état d'équilibre entre prolifération, différenciation et apoptose dans des situations où cet équilibre est perturbé.

Il est préférable de considérer comme efficaces sur LOX les principes actifs permettant d'obtenir une différence d'environ $\pm$ 50% de l'expression de l'ARNm de LOX et/ou une différence d'environ $\pm$ 15 % de l'expression de LOX et/ou de l'activité de LOX sur un modèle, comprenant au moins un type cellulaire présentant une expression et/ou une activité de LOX, au contact de ces principes actifs par rapport au niveau d'expression et/ou d'activité de LOX dans un modèle témoin (généralement sans mise au contact des principes actifs).

Il est préférable de considérer comme efficaces sur NRAGE les principes actifs permettant d'obtenir une différence d'environ $\pm$ 50% de l'expression de l'ARNm de NRAGE et/ou une différence d'environ $\pm$ 15 % de l'expression de NRAGE et/ou de l'effet biologique de NRAGE sur un modèle, comprenant au moins un type cellulaire présentant une expression et/ou une activité de NRAGE, au contact de ces principes actifs par rapport au niveau d'expression et/ou d'activité de NRAGE dans un modèle témoin (généralement sans mise au contact des principes actifs). C'est ainsi que la présente invention concerne selon un premier aspect l'utilisation selon la revendication 1. Avantageusement, l'expression de LOX est stimulée dans des cellules épithéliales, en particulier dans des kératinocytes.

La substance est destinée au traitement et/ou la prévention du vieillissement de la peau.

Avantageusement, la substance est destinée à diminuer l'apoptose au niveau de l'épiderme en cas de forte apoptose au cours du vieillissement de la peau.

Avantageusement, la substance est destinée à augmenter la prolifération cellulaire en cas d'hypoprolifération cellulaire au niveau de l'épiderme au cours du vieillissement.

La substance est donc destinée à stimuler l'expression de LOX, et éventuellement inhiber l'expression de NRAGE, au cours du vieillissement de la peau.

Ladite composition est une composition cosmétique.

Avantageusement, l'équilibre cellulaire entre la prolifération, la différenciation, et l'apoptose est l'équilibre entre la prolifération, la différenciation, et l'apoptose des kératinocytes.

Avantageusement, la matière première utilisée pour la préparation du principe actif, est stérilisée ou non par rayonnement, par exemple bêta ou gamma à une dose de préférence de 5kGy puis est réduite en poudre si nécessaire, par exemple par broyage à température ambiante. La poudre est ensuite par exemple dispersée à raison de 2 à 5% (poids/poids) de poudre, de préférence 5%, soit dans un solvant polaire, par exemple eau, alcool, glycol, comme le butylène glycol, ou polyol, et/ou dans un mélange de solvants polaires, avantageusement un mélange eau/(alcool, glycol ou polyol) (tels que éthanol, glycérol, butylène glycol et autres glycols, xylitol etc.) en proportions variables et de préférence dans un mélange eau/butylène glycol 75/25 ou 50/50, soit dans un solvant apolaire, comme par exemple un alcane, soit dans un mélange de solvants apolaires, soit dans un mélange de solvants polaires et apolaires. Après de préférence une agitation pendant au minimum 2 heures, par exemple magnétique, et éventuellement chauffage du solvant, l'échantillon est de préférence clarifié par décantation ou centrifugation puis filtré de préférence à 0,45µm ou 0,22µm.

Avantageusement, le principe actif obtenu selon l'une des méthodes décrites ci-dessus est utilisé à une concentration finale de préférence comprise entre 0,01% volume/volume (v/v) et 10 % (v/v) et de préférence comprise entre 0,1% et 1% (v/v).

Ladite substance est un extrait de bois total de Quassia de Surinam (Cassia amara).

L'invention concerne selon un autre aspect un procédé de préparation d'une composition comprenant :

- le mélange du principe actif avec au moins un excipient pour réaliser une composition cosmétique, destinée à prévenir ou à traiter au moins un état dans lequel l'équilibre cellulaire entre la prolifération, la différenciation, et l'apoptose est absent ou altéré.

Avantageusement, les kératinocytes sont des kératinocytes humains.

### Description détaillée de l'invention:

Les inventeurs ont découvert de façon inattendue une interaction entre LOX et la protéine NRAGE. Cette découverte est capitale et elle est le point de départ de la présente invention. Elle désigne en effet LOX comme étant la protéine qui, située à la croisée des voies de la prolifération, de la différenciation et de l'apoptose, est capable de contrôler l'homéostasie cellulaire.

D'autre part, les inventeurs ont découvert que la protéine NRAGE est présente en particulier au niveau de l'épiderme et exprimée en particulier par les kératinocytes. Cette découverte permet également de faire de l'interaction entre LOX et NRAGE une cible d'action de soins cosmétiques ou de traitements destinés à restaurer l'homéostasie cellulaire, dans les situations dans lesquelles elle est perturbée.

### 1) Découverte et caractérisation de l'interaction LOX/NRAGE *in vitro*

Ayant préalablement découvert la présence de LOX dans l'épiderme (Noblesse et al, Lysyl oxydase-like and lysyl oxydase are present in the dermis and epidermis of a skin equivalent and in human skin and are associated to elastic fibers, J. Invest. Dermatol. 122 :621-630, 2004), les inventeurs ont cherché à déterminer le(s) rôle(s) que LOX pouvait jouer à ce niveau, sachant que le rôle principal que l'on lui connaît aujourd'hui - réticulation du collagène et de l'élastine - était peu vraisemblable dans ces cellules qui ne font pas (ou peu) de collagène et d'élastine au niveau où LOX a été localisée.

Dans cet objectif, ils ont recherché les partenaires protéiques de LOX par la technique du double hybride dans la levure, une banque d'ADNc de kératinocytes de peau humaine normale a été criblée, en utilisant en particulier l'appât GAL4 BD-hL0Xmat, permettant ainsi de mettre en évidence NRAGE comme partenaire potentiel de LOX (voir exemple 1).

Les inventeurs ont ensuite vérifié, par la technique du double hybride dans les cellules Hela, que ce partenariat potentiel s'observait également dans les cellules de mammifères (voir exemple 2).

L'étape suivante a consisté à déterminer si l'identification de ce partenariat potentiel entre LOX et NRAGE pouvait correspondre à une interaction physique, directe, entre ces deux protéines. Ceci a été réalisé en particulier par une technique de co-immunoprécipitation des protéines LOX et NRAGE produites après transfection de leurs gènes dans des cellules Cos7. Les résultats obtenus ont, d'une part, confirmé l'interaction physique entre LOX et NRAGE et, d'autre part, permis de préciser que LOX interagissait avec une région particulière de NRAGE, appelée IRD (Interspersed Repeat Domain), qui est une région spécifique à NRAGE dans la famille des protéines MAGE (voir exemple 3).

Dans ce cadre, il faut noter à ce stade que la découverte de l'interaction entre LOX et NRAGE revêt un intérêt tout particulier quand on considère de façon conjuguée que, d'une part, la protéine NRAGE contient deux domaines riches en résidus lysyls, qui pourraient être des sites propices à sa dimérisation sous l'action catalytique de LOX et que, d'autre part, c'est précisément sous la forme de dimère que semble s'exercer la fonctionnalité de NRAGE.

L'existence, *in vitro,* d'une interaction physique, directe, entre LOX et NRAGE, localisée au niveau de la région IRD de NRAGE, ayant ainsi été découverte, les inventeurs se sont posés la question de savoir si cette interaction intervenait également *in vivo.* Il est essentiel de rappeler à ce stade que, contrairement à celle de LOX, la présence de NRAGE au niveau de la peau n'avait jamais été décrite auparavant.

### 2) Mise en évidence de la présence de NRAGE dans la peau humaine normale et dans la peau humaine normale reconstruite

Les inventeurs ont mis en évidence l'expression de NRAGE dans la peau au niveau, à la fois, du derme et de l'épiderme (voir exemples 4 et 5).

Dans le cadre de la présente invention, les inventeurs ont mis en oeuvre une méthode de localisation de l'expression de NRAGE dans la peau.

Au niveau de l'épiderme, les inventeurs ont mis en évidence, de manière inattendue, que NRAGE n'était pas exprimé de façon homogène, ce que l'on aurait pu attendre d'une protéine dont la présence est généralement décrite comme ubiquitaire, mais sous forme d'un gradient d'expression.

Rappel sur la structure de l'épiderme:

L'épiderme est un épithélium stratifié reposant sur une membrane basale qui l'ancre au derme. Son épaisseur varie de 60 à 100 $\mu$m en moyenne et il est constitué de 4 couches continues, de la profondeur vers la surface, résultant d'une différenciation progressive des kératinocytes:

- la couche basale (rangée unique de cellules)
- la couche épineuse (5 à 6 assises cellulaires)
- la couche granuleuse (1 à 3 assises cellulaires)
- la couche cornée ou squameuse (5 à 10 assises cellulaires)

Ainsi NRAGE est absent dans la couche basale et apparaît au niveau des premières couches de kératinocytes différenciés, le marquage augmentant dans les couches squameuses. Le marquage des premières couches suprabasales de l'épiderme correspond à celui observé pour LOX, LOX apparaissant dès la couche basale. En revanche, le marquage de LOX diminue dans les couches supérieures de l'épiderme, où NRAGE est présent, d'où la distinction de 3 zones:

- 1 zone inférieure de l'épiderme comprenant la couche basale et les premières suprabasales prolifératives où seule LOX est exprimée ;
- 1 zone intermédiaire allant des premières couches suprabasales non-prolifératives aux premières assises squameuses dans laquelle LOX et NRAGE sont co-exprimées ;
- 1 zone supérieure de l'épiderme où seul NRAGE est exprimée.

Les observations au niveau cellulaire montre que LOX et NRAGE apparaissent à la périphérie de la cellules, en particulier en région sous-membranaire, NRAGE apparaissant également au niveau du cytoplasme. (voir exemples 4 et 5)

Les inventeurs ont ainsi démontré, pour la première fois, la présence de NRAGE au niveau de la peau, dans le derme et dans l'épiderme. Ils ont également montré que l'épiderme comporte une partie dans laquelle les deux protéines LOX et NRAGE sont exprimées et partagent, au niveau cellulaire, notamment au niveau des kératinocytes, la même localisation: la zone périphérique sous-membranaire (NRAGE étant en outre également présent au niveau cytoplasmique).

Les inventeurs ont mis ainsi en évidence que les conditions étaient réunies pour que l'interaction directe qu'ils ont mise en évidence *in vitro* puissent s'opérer dans l'épiderme au niveau de la zone de co-localisation de LOX et de NRAGE.

### 3) Mise en évidence de perturbations de l'expression de LOX et/ou de NRAGE au niveau de l'épiderme avec l'age et dans certaines situations pathologiques

Les inventeurs ont également mis en évidence de façon inattendue que le vieillissement cutané ainsi qu'un certain nombre de situations pathologiques sont accompagnées de perturbations de l'expression de LOX et/ou de NRAGE au niveau de l'épiderme.

### 3.1) Etat de LOX et de NRAGE dans l'épiderme

### 3.1.1) Peau de personnes âgées

La peau des personnes âgées est caractérisée par un épiderme hypoprolifératif, très fin (réduit à quelques couches cellulaires), et hyperkératinisé.

Les inventeurs y ont mis en évidence la totale absence de LOX (détectable par les techniques utilisées). En revanche, NRAGE est fortement exprimée, localisée au niveau du cytoplasme, et apparaît dès la première couche suprabasale, sans gradient d'expression.

Ainsi, la présente invention permet de stimuler l'expression (et/ou l'activité) de LOX, avec ou sans inhibition de l'expression (et/ou activité) de NRAGE, de façon à ré-induire une zone de différenciation et/ou une zone d'apoptose régulée, notamment par la restauration d'un zone de co-expression de LOX et de NRAGE. En outre, on obtient ainsi un épaississement de peau, notamment de l'épiderme.

Ainsi, la présente invention est destinée notamment à corriger les effets du vieillissement de la peau, ou à les prévenir.

### 3.1.2) réaction du greffon contre l'hôte - GVH (Graft Versus Host)

La GVH est une maladie qui peut survenir à la suite d'allogreffes de cellules souches hématopoïétiques. Elle est liée à l'effet des cellules immunitaires (lymphocytes) contenues dans le greffon contre les organes normaux du patient (notamment la peau, le foie et le tube digestif). Au niveau de la peau, elle se manifeste par une éruption maculopapuleuse, prurigineuse et inflammatoire.

Les patients atteints de cette maladie ont une peau très fine, avec un épiderme fortement apoptotique.

L'étude histologique réalisée par les inventeurs a mis en évidence l'absence de LOX et une présence très marquée de NRAGE, localisée au niveau cytoplasmique et apparaissant dès les premières couches suprabasales.

### 3.1.3) Le lichen plan

Le lichen est une maladie de la peau, de cause inconnue, caractérisée par la présence de papules de quelques millimètres de diamètre, violettes, aplaties, fermes, sèches, très prurigineuses.

Les inventeurs ont mis en évidence une très forte diminution de expression de LOX, voire son absence totale, ainsi qu'une expression très irrégulière de NRAGE. Ces observations traduisent certainement un niveau d'interaction inexistant, ou très faible, entre les deux protéines étudiées.

### 3.1.4) Psoriasis

Le psoriasis est une maladie chronique de la peau, caractérisée par des lésions érythémato-squameuses. L'élément fondamental en est une augmentation de la vitesse de multiplication des kératinocytes, responsable d'un renouvellement plus rapide et d'un épaississement de l'épiderme.

Au niveau histologique, on observe une hyper-prolifération des cellules engagées dans les premières étapes de différenciation terminale, une différenciation terminale incomplète, à laquelle s'associe une apoptose absente ou très peu marquée.

Les inventeurs ont détecté une expression très forte de LOX et une présence moyenne de NRAGE, avec un marquage plus ou moins homogène des zones concernées, ne montrant pas de gradient d'expression. A ces caractéristiques d'intensité d'expression variant de la normale, s'ajoutent des anomalies beaucoup plus importantes touchant à la localisation des protéines concernées. Ainsi, dans les peaux psoriatiques, LOX est exprimée essentiellement dans la partie inférieure de l'épiderme et NRAGE uniquement dans sa partie supérieure, l'expression des protéines étant ainsi décalée, sans zone de recouvrement, telle qu'elle est normalement observée dans la peau saine. Au niveau cellulaire, NRAGE est observée au niveau cytoplasmique uniquement et pas en zone périphérique sous-membranaire.

Ces observations montrent que dans le psoriasis, bien que les protéines LOX et NRAGE soient toutes les deux présentes dans l'épiderme, elles ne peuvent interagir de façon directe l'une et avec l'autre puisqu'elles ne sont pas physiquement présentes au même endroit. Cette absence d'interaction se traduit par des dysfonctionnements observés au niveau des épidermes, reflétant ainsi la rôle de l'interaction entre LOX et NRAGE dans le maintien de l'homéostasie épidermique.

Ainsi, l'inhibition de l'expression (et/ou activité) de LOX, et/ou éventuellement la stimulation, de préférence partielle, de l'expression (et/ou l'activité) de NRAGE permet notamment d'obtenir une zone de recouvrement de l'expression de LOX et de NRAGE pour ré-induire une zone de prolifération régulée permettant de diminuer l'hyperprolifération en favorisant l'apoptose au niveau des kératinocytes.

D'autre part, la stimulation de l'expression (et/ou l'activité) de NRAGE et/ou l'inhibition, de préférence partielle, de l'expression (et/ou activité) de LOX, permet notamment d'obtenir une zone de recouvrement de l'expression de LOX et de NRAGE pour ré-induire une zone de prolifération régulée permettant de diminuer l'hyperprolifération en favorisant l'apoptose au niveau des kératinocytes.

### 3.1.5) Eczéma

L'eczéma est une affection de la peau qui se caractérise cliniquement par des rougeurs, des gonflements localisés plus ou moins étendus, ainsi que des vésicules suintantes formant ensuite des croûtes, accompagnées de fortes démangeaisons. Dans sa phase chronique l'eczéma se complique d'une modification de la peau avec épaississement. Au niveau de l'épiderme, l'apoptose est réduite.

Les inventeurs ont mis en évidence une très forte expression de LOX, localisée à la périphérie des cellules. En revanche, NRAGE est peu exprimée et est observée au niveau cytoplasmique uniquement, ce qui traduit certainement un niveau d'interaction inexistant, ou très faible, entre les deux protéines étudiées.

Ainsi, la stimulation de l'expression (et/ou l'activité) de NRAGE, et/ou l'inhibition, de préférence partielle, de l'expression (et/ou activité) de LOX, permet d'augmenter l'apoptose et de réduire ainsi certains effets de l'eczéma.

### 3.1.6) Les cancers cutanés épithéliaux

On distingue deux grands types de carcinomes cutanés épithéliaux:

- le carcinome basocellulaire (90 % des cas) est une tumeur d'évolution lente, essentiellement locale, qui ne métastase quasiment jamais. Il résulte d'une prolifération incontrôlée des kératinocytes de la couche basale.
- le carcinome spinocellulaire (10 % des cas) a une évolution locale beaucoup plus agressive et peut métastaser. Il a pour origine une prolifération incontrôlée des kératinocytes de la couche spineuse.

Les inventeurs ont mis en évidence l'absence de LOX et de NRAGE dans les cellules invasives des deux types de cancers étudiés (cancers basocellulaires et spinocellulaires) avec une perte progressive de LOX et de NRAGE dans l'épiderme au voisinage des tumeurs. Les inventeurs ont également observé que LOX est fortement exprimée dans la réaction stromale autour des tumeurs, alors que NRAGE en est absent.

On peut noter à ce stade que la perte d'expression de LOX, qui n'avait jamais été mise en évidence précédemment dans les cas de ces deux types de cancers, est inattendue et, peut-être, spécifique aux cancers épithéliaux, puisque LOX est généralement considérée comme étant présente dans les cancers *in situ.*

La perte d'expression de NRAGE est elle aussi complètement nouvelle, et n'a jamais été décrite dans aucun cancer.

Ainsi, la stimulation de l'expression (et/ou l'activité) de NRAGE, et de l'expression (et/ou activité) de LOX, permet de restaurer l'homéostasie. Il est possible de réverser le phénotype tumoral, notamment au niveau des cancers cutanés épithéliaux.

### 3.2) Conclusion des études sur tissus pathologiques

A partir de ces observations, les inventeurs ont pu mettre en évidence que les situations dans lesquelles intervient une dérégulation de l'équilibre entre prolifération, différenciation et apoptose, que ce soit au niveau de l'épiderme de sujets âgés, ou de sujets atteints de différentes pathologies affectant notamment l'épiderme, étaient caractérisées de façon systématique par des défauts d'expression de LOX et/ou de NRAGE, lesquels défauts étant de nature à altérer ou à rendre impossible leur interaction.

Ce constat a conduit les inventeurs à rechercher le moyen de restaurer l'équilibre entre prolifération, différenciation et apoptose en utilisant le contrôle exercé par LOX ou NRAGE, de préférence en utilisant le couple LOX-NRAGE.

Ainsi, à partir de ces découvertes inattendues, les inventeurs ont réalisé des méthodes d'identification de principes actifs modulant l'expression de LOX et/ou de NRAGE pour restaurer le contrôle exercé par ces protéines *via* leur interaction, dans le but d'identifier des principes actifs pour réaliser des compositions notamment cosmétiques ou pharmaceutiques.

De cette façon, dans les cas d'hypo-prolifération épidermique (peaux âgées, GVH), caractérisé par un épiderme extrêmement fin et l'absence d'expression de LOX, on stimule l'expression de LOX, avec ou sans inhibition de l'expression de NRAGE, en vue de ré-induire, à travers la modulation de la co-expression de LOX et de NRAGE, une zone de différenciation et d'apoptose régulée, qui conduit à un épaississement de la peau.

Dans les cas d'hyper-prolifération épidermique (psoriasis, eczéma), dans lesquels NRAGE est sous-exprimée, on stimule l'expression de NRAGE, avec ou sans inhibition (légère) de celle de LOX, de façon à favoriser l'apoptose en ré-induisant une zone de prolifération régulée (recouvrement LOX/NRAGE) et en permettant de stopper l'hyper-prolifération.

Dans les cas de forte apoptose au niveau de l'épiderme (peaux âgées, exposition de la peau à un stress, en particulier exposition à la chaleur, ou exposition de la peau à un rayonnement, en particulier un rayonnement solaire, ou exposition de la peau à un agent toxique, par exemple chimique ou microbiologique, ou de la maladie de la réaction du greffon contre l'hôte - GVH), dans lesquels NRAGE est sur-exprimée, on inhibe l'expression de NRAGE avec ou sans stimulation de l'expression de LOX.

## 4) Mise en évidence de l'implication de LOX dans l'apoptose cellulaire

De façon inattendue, les inventeurs ont mis en évidence l'existence d'un lien encore inconnu entre LOX et l'apoptose. Ainsi, les données obtenues reflètent le rôle anti-apoptotique de LOX dans les kératinocytes, lequel rôle fait intervenir la régulation de la protéine pro-apoptotique NRAGE, notamment.

## 5) Recherche de principes actifs

La mise en évidence des principes actifs a été fait notamment par l'analyse de l'expression des ARN messagers de LOX et de NRAGE, en particulier sur des kératinocytes en culture, de préférence des kératinocytes humains. Les principes actifs dont l'activité est à tester sont placés en contact avec les kératinocytes en culture pendant une durée suffisante et dans des conditions adéquates pour que le contact soit efficace. Les principes actifs sont de préférence testés dans différentes concentrations pour éventuellement détecter l'influence de la concentration.

Avantageusement, les principes actifs criblés sont d'origine végétale, notamment pour éviter les problèmes liés à la synthèse chimique. Les avantages des principes actifs d'origine végétale sont bien connus de l'homme de l'art, notamment en pharmacie, en dermo-pharmacie, en neutraceutique, et en cosmétique.

La recherche de principes actifs est effectuée notamment en réalisant l'extraction des ARN totaux puis en effectuant une RT-PCR quantitative. En particulier, les séquences d'amorces préférées sont celles utilisées dans l'exemple 13 sans y être limité.

La quantité d'ADNc de chaque essai est rapportée à la quantité d'ADNc de l'actine. On compare ensuite l'effet de la présence ou non de principes actifs. Lorsque l'expression et/ou l'activité de NRAGE et/ou LOX sont modulées (stimulées ou inhibées) par rapport aux témoins, on peut alors qualifier la substance de principe actif. Avantageusement, un principe actif permet de moduler d'au moins 50 % l'expression de l'ARN messager de LOX et/ou de NRAGE, ou d'au moins 15 % l'expression et/ou de l'activité de LOX et/ou NRAGE.

Le composé selon la présente invention est préparé sous forme de composition cosmétique. De ce fait, pour cette composition, l'excipient contient par exemple au moins un composé choisi parmi le groupe consistant en les conservateurs, les émollients, les émulsifiants, les tensioactifs, les hydratants, les épaississants, les conditionneurs, les agents matifiant, les stabilisants, les antioxydants, les agents de texture, les agents de brillance, les agents filmogènes, les solubilisants, les pigments, les colorants, les parfums et les filtres solaires. Ces excipients sont de préférence choisis parmi le groupe consistant en les acides aminés et leurs dérivés, les polyglycérols, les esters, les polymères et dérivés de cellulose, les dérivés de lanoline, les phospholipides, les lactoferrines, les lactoperoxidases, les stabilisants à base de sucrose, les vitamines E et ses dérivés, les cires naturelles et synthétiques, les huiles végétales, les triglycérides, les insaponifiables, les phytosterols, les esters végétaux, les silicones et ses dérivés, les hydrolysats de protéines, l'huile de Jojoba et ses dérivés, les esters lipo/hydrosolubles, les betaines, les aminoxides, les extraits de plantes, les esters de saccharose, les dioxydes de titane, les glycines, et les parabens, et encore de préférence parmi le groupe consistant en le butylène glycol, le stéareth-2, le stéareth-21, le glycol-15 stéaryl éther, le cétéaryl alcool, le phénoxyéthanol, le méthylparaben, l'éthylparaben, le propylparaben, le butylparaben, le butylène glycol, les tocopherols naturels, la glycérine, le sodium dihydroxycétyle, l'isopropyl hydroxycétyl éther, le glycol stéarate, le triisononaoin, l'octyl cocoate, le polyacrylamide, l'isoparaffine, le laureth-7, un carbomer, le propylène glycol, le glycérol, le bisabolol, une diméthicone, l'hydroxyde de sodium, le PEG 30-dipolyhydroxystérate, les caprique/caprylique triglycérides, le cétéaryl octanoate, le dibutyl adipate, l'huile de pépin de raisin, l'huile de jojoba, le sulfate de magnésium, l'EDTA, une cyclométhicone, la gomme de xanthane, l'acide citrique, le lauryl sulfate de sodium, les cires et les huiles minérales, l'isostéaryl isostéarate, le dipélargonate de propylène glycol, l'isostéarate de propylène glycol, le PEG 8 Beewax, les glycérides d'huile de coeur de palme hydrogénée, les glycérides d'huile de palme hydrogénée, l'huile de lanoline, l'huile de sésame, le cétyl lactate, le lanoline alcool, l'huile de ricin, le dioxyde de titane, le lactose, le saccharose, le polyéthylène basse densité, une solution isotonique salée.

Avantageusement, la composition précitée est formulée sous une forme choisie parmi le groupe consistant en une solution, aqueuse ou huileuse, une crème ou un gel aqueux ou un gel huileux, notamment en pot ou en tube, notamment un gel douche, un shampoing ; un lait ; une émulsion, une microémulsion ou une nanoémulsion, notamment huile-dans-eau ou eau-dans-huile ou multiple ou siliconée ; une lotion, notamment en flacon de verre, de plastique ou en flacon doseur ou en aérosol ; une ampoule ; un sirop ; un savon liquide ; un pain dermatologique ; une pommade ; une mousse ; une solution injectable ; un produit anhydre, de préférence liquide, pâteux ou solide, par exemple sous forme de bâtonnet, notamment sous forme de rouge à lèvre ; une poudre ; un comprimé.

Sur les figures :

- La figure 1 représente une séquence et un schéma de la protéine NRAGE;
- La figure 2 représente la moyenne des résultats obtenus à l'exemple 2 pour l'interaction en double hybride ;
- La figure 3 représente la mise en évidence de la présence de LOX et de NRAGE dans la peau reconstruite ;
- La figure 4 représente la localisation en microscopie confocale de LOX et NRAGE sur des coupes de peau humaine ;
- La figure 5 représente une détection de LOX et NRAGE sur des coupes de peau humaine d'un donneur de 91 ans ;
- La figure 6 représente la détection de LOX et de NRAGE dans la peau d'une personne atteinte de la maladie de réponse du greffon contre l'hôte ;
- La figure 7 représente la détection de LOX et de NRAGE dans la peau d'un patient présentant un cancer de type basocellulaire ou spinocellulaire. ;
- La figure 8 représente la détection de LOX et de NRAGE dans la peau d'un patient atteint de lichen plan ;
- La figure 9 représente la mise en évidence de la localisation de LOX et de NRAGE dans la peau d'un patient atteint de psoriasis ;
- La figure 10 représente la mise en évidence de la localisation de LOX et de NRAGE dans la peau d'un patient atteint d'eczéma.
- La figure 11 présente le marquage globale de la peau reconstruite au bleu Evans (couches épidermique en rouge, cellules en bleu, fibres du substrat dermique en rouge, jonction dermo-épidermique en pointillés).
- La figure 12 présente la détection immuno-histochimique de la cyto-kératine 10 sur la peau reconstruite (cellules exprimant fortement le marqueur en rouge, noyaux en bleu, jonction dermo-épidermique en pointillés).
- La figure 13 présente la détection immuno-histochimique de la transglutaminase sur la peau reconstruite (cellules exprimant fortement le marqueur en rouge, noyaux en bleu, jonction dermo-épidermique en pointillés).

D'autres buts, caractéristiques et avantages de l'invention apparaîtront clairement à l'homme de l'art suite à la lecture de la description explicative qui fait référence à des exemples qui sont donnés seulement à titre d'illustration et qui ne sauraient en aucune façon limiter la portée de l'invention.

Les exemples font partie intégrante de la présente invention et toute caractéristique apparaissant nouvelle par rapport à un état de la technique antérieure quelconque à partir de la description prise dans son ensemble, incluant les exemples, fait partie intégrante de l'invention dans sa fonction et dans sa généralité.

Ainsi, chaque exemple a une portée générale.

D'autre part, dans les exemples, tous les pourcentages sont donnés en poids, sauf indication contraire, et la température est exprimée en degré Celsius sauf indication contraire, et la pression est la pression atmosphérique, sauf indication contraire.

### EXEMPLES

### Exemple 1: clonage de NRAGE par technique de double hybride chez la levure.

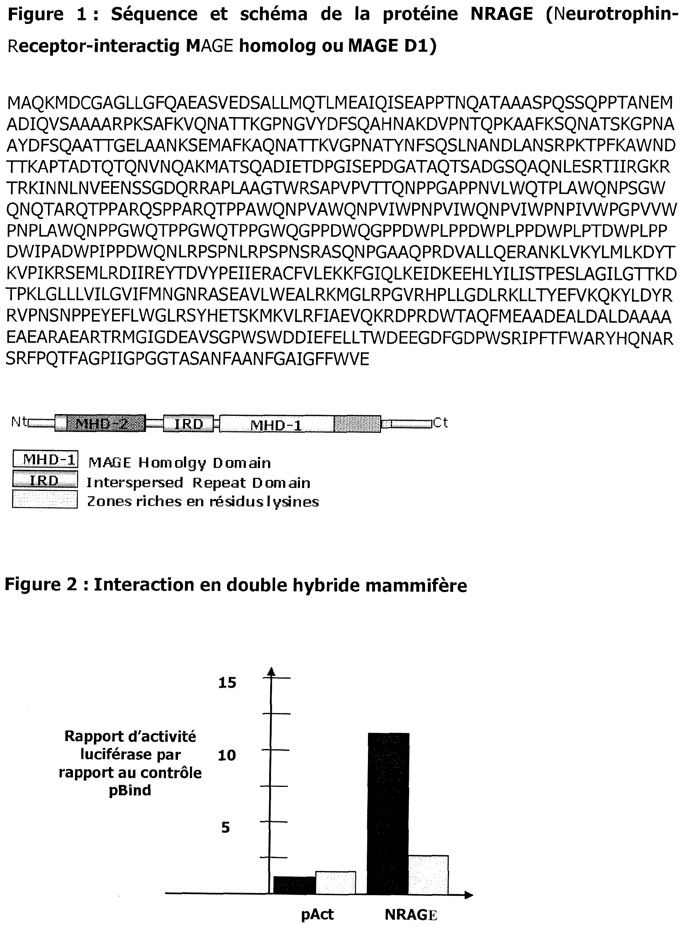

Il a initialement consisté à rechercher les partenaires potentiels de LOX dans les kératinocytes. La technique de double hybride chez la levure (2HL) a été employée. Le système 2HL permet l'identification et la caractérisation d'interactions entre une protéine "appât" et des partenaires potentiels "cibles". En l'occurrence, l'appât a été la région mature de LOX fusionnée au domaine de liaison à l'ADN (Binding Domain, BD) du gène Gal4. La cible ou les cibles ont été les gènes codés par une banque d'ADN complémentaire de kératinocytes humains, fusionnés au domaine d'activation (Activation Domain, AD) du gène Gal4. L'interaction des domaines de l'appât (LOX) avec les domaines codés par une séquence de la banque permet la liaison et l'activation du promoteur Gal4, lequel contrôle différents gènes conférant l'auxotrophie aux levures AH109, permettant la croissance sur milieu déficient et l'activation des activités galactosidases. Un gène codant pour une protéine candidate à être partenaire de LOX a ainsi été mis en évidence, grâce à cette technique de sélection possible d'interactants : la protéine intracellulaire Melanoma Associated Antigen D1 (MAGE-D1) ou NRAGE (Figure 1).

### Screening d'une librairie de kératinocytes en double hybride levure par l'appât GAL4 BD-hLOXmat (human LOX mature).

La librairie de cDNA de kératinocytes de peau humaine normale dans le vecteur pGAD-10 a été utilisée. L'appât choisi pour ce criblage a été LOXmat, codée par la région de l'ADNc humain de l'enzyme LOX sans le peptide signal ni la pro-région. La séquence nucléotidique a été insérée en phase derrière le domaine de liaison à l'ADN (Binding Domaine : BD) du facteur de transcription GAL4 dans le vecteur pBD-Gal4 Cam. Le fragment LOX +494 à +1254 à insérer a été amplifié par PCR (polymerase chain reaction), en derrière le binding-domain de Gal4 dans un vecteur pGal4-BD à l'aide des amorces suivantes :

1) ggg atc cgc atg gtg ggc gac gac (introduisant Bam HI) (SEQ ID N° 11)

2) ttg tcg act aat acg gtg aaa ttg tgc (introduisant Sal I au niveau du stop qui est conservé) (SEQ ID N° 12).

Le fragment amplifié a initialement été introduit dans le vecteur TOPO, puis inséré dans pGal4-BD. Pour valider l'expression de la protéine de fusion BD-LOXmat, les levures AH 109 ont été transformées par le plasmide appât pBD-LOXmat.

On effectue le criblage des levures transformées: $6,88 \times 10^6$ cfu (croissance en milieu déficient) dans la levure AH 109, 80 clones sélectionnés poussant en milieu déficient en adénine, histidine, tryptophane et leucine, dont 23 ont été retenus pour leur forte croissance.

### Exemple 2: validation de l'interaction entre LOX et NRAGE par technique de double hybride chez le mammifère (2HM).

Les cellules Hela sont transfectées d'une part par les plasmides pAct et pAct-NRAGE, et d'autre part par pBind et pBind-LOXmat en présence de lipofectamine. L'activité luciférase est testée après 48h, et les résultats sont exprimés en rapport d'activité luciférase par rapport au contrôle (vecteur vide pBind). Les expériences sont effectuées en triple, la moyenne des résultats obtenus est représentée sur la Figure 2.

La séquence hLOX utilisée dans l'exemple 1 pour le double hybride levure a été insérée en phase derrière le binding-domain de Gal4 dans le vecteur pBind (Promega, Madison USA)) pour les interactions double hybride mammifère.

La proie N-RAGE a été insérée derrière l'activating domain VP16 Gal4 dans le vecteur pAct (Promega). Elle débute à l'acide aminé 152 (nucléotide 458).

### Exemple 3: co-immunoprécipitation de LOX et de NRAGE dans les cellules de mammifères.

Les cellules Cos7 (cellules épithéliales de rein de mammifère) sont co-transfectées pour les gènes LOX (complet humain, région mature humaine et région mature murine) par les constructions pcLOX32-V5His (LOX), ou, pcLOX36-V5His ou pcLOX-27-V5His et pour le gène NRAGE par pNM3-HA (NRAGE complet) ou pNM7-HA (région IRD). La transfection se fait dans des boites de Pétri de diamètre 100 mm en utilisant la lipofectamine. Après 48h, les cellules transfectées sont lysée dans $500\mu$L de tampon de lyse, Les protéines des lysats cellulaires sont incubées soit avec l'anti-V5 soit avec l'anti-HA ; l'anti-corps monoclonal anti-V5 ou anti-HA est ajouté au 1/250 au lysat cellulaire pendant 1h30 sous agitation à 4°C.

Les complexes immuns (CI) ainsi formés sont précipités par la protéine G-sépharose sous agitation à 4°C pendant 1h. Après trois lavages de 10 min en tampon de lyse et une élution en tampon SDS-PAGE, une électrophorèse de la totalité des CI est effectuée sur un gel 10% SDS-PAGE suivi d'un Western blot. Après transfert des CI sur membrane PVDF (fluorure de polyvinylidène), la révélation est réalisée:

- pour NRAGE, avec l'anti-HA dilué au 1/1.000 puis l'anti-souris-HRP (horse radish peroxidase) dilué au 1/20.000
- pour LOX, par l'anti-V5-HRP dilué au 1/5.000,

La détection finale est réalisée par chimioluminescence de la HRP.

Les résultats obtenus démontrent que la forme complète de NRAGE (NM3-HA) co-immunoprécipite avec la forme entière humaine de LOX (LOX 32H), de LOX humaine mature (LOX 36H) et de LOX murine mature (LOX 27H). De plus, NRAGE (NM7-HA), qui correspond à la région IRD, co-immunoprécipite de la même façon avec ces trois protéines recombinantes, montrant l'implication de cette région dans l'interaction.

### Exemple 4: mise en évidence, par immuno-histochimie, de la présence de LOX et de NRAGE dans un modèle de peau reconstruite.

La mise en évidence a été réalisée dans un modèle de peau reconstruite (MIMESKIN®, Engelhard Lyon, France) préparé à partir d'un substrat dermique (collagène/glycosaminoglycannes/chitosane, MIMEDISC®, Engelhard Lyon, France) ensemencé par des fibroblastes humain normaux, à la surface duquel ont été déposés des kératinocytes humains normaux.

Après 45 jours de culture, permettant la différenciation des kératinocytes par exposition à l'interface air-liquide, les échantillons sont fixés dans le fixateur de Bouin ou dans le formaldéhyde, puis inclus en paraffine.

Les immuno-marquages sur coupes sont réalisés avec les anticorps décrits ci-dessous :

- anticorps anti-LOX obtenu et purifié selon la méthode décrite par Sommer et al. (Transient expression of lysyl oxidase by liver myofibroblasts in murine schistosomiasis, Laboratory Investigation 69:460-470, 1993)

- anticorps anti-NRAGE (goat polyclonal IgG),
- anticorps secondaire de lapin anti-chèvre.

Les complexes immuns sont détectés avec une IgG anti-lapin conjuguée à la peroxydase, en utilisant la diaminobenzidine comme substrat, puis en effectuant une contre-coloration à l'hématoxyline.

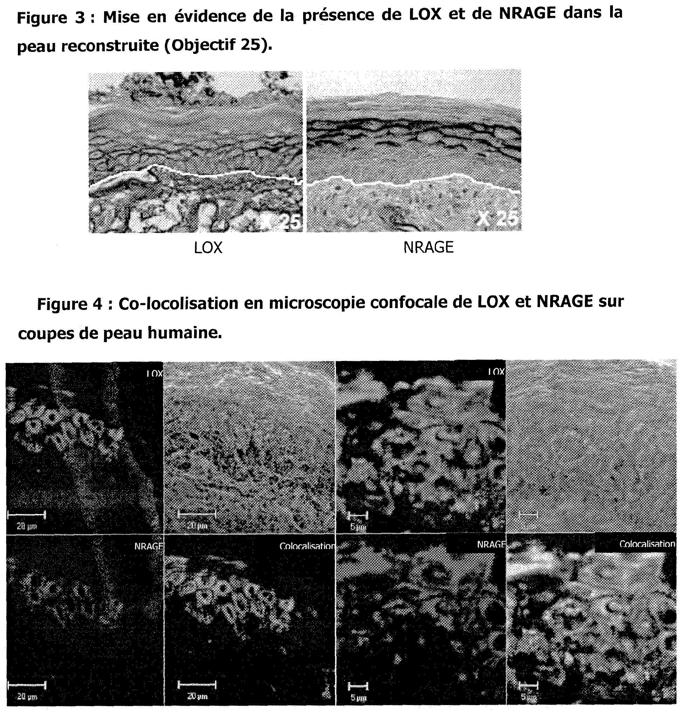

La **Figure 3** présente la détection immuno-histochimique de LOX et NRAGE sur la peau reconstruite.

La position de la jonction dermo-épidermique est indiquée par une ligne continue, celle du substrat dermique avec une flèche, et la localisation des kératinocytes est indiquée avec une tête de flèche.

L'expression de LOX apparaît dès la couche basale, persiste dans les couches suprabasales, et disparaît progressivement dans les couches différenciées. L'expression de NRAGE est légèrement décalée, elle s'affirme dès les couches suprabasales non prolifératives et s'intensifie fortement dans les couches granuleuses et squameuses, là ou LOX n'est plus exprimée.

Elles sont donc sont associés dans les couches suprabasales non prolifératives de l'épiderme du modèle de peau reconstruite MIMESKIN®. Cette co-localisation a été confirmée en immuno-histologie (IH) sur la peau humaine normale.

Les inventeurs ont ainsi mis en évidence que les protéines LOX et NRAGE sont exprimées dans l'épiderme, avec une zone de co-localisation au niveau des couches spineuses.

**Exemple 5: mise en évidence de la co-localisation de LOX et de NRAGE dans la peau humaine normale en microscopie confocale.**

Des coupes congelées d'échantillons de peau humaine normale issus de résection chirurgicale (prépuce) sont réalisées. Les anticorps primaires anti-LOX et anti-NRAGE de l'exemple 4 ont été utilisés pour détecter l'expression de LOX et NRAGE. Les anticorps secondaires utilisés sont:

- Âne anti-lapin IgG-F1TC, marquage fluorescéine en vert ;
- Âne anti-chèvre IgG-R, marquage rhodamine en rouge.

Un témoin négatif a été réalisé en absence d'anticorps primaires.

L'observation du double marquage a été faite en utilisant un microscope droit confocal AXIOPLAN 2 LSM510 ZEISS, et l'acquisition des images a été faite en utilisant le logiciel d ZEISS LSM5 Image Browser.

La **Figure 4** présente l'immuno-détection de LOX et de NRAGE dans la peau humaine normale en microscopie confocale.

Les observations confirment les résultats de l'exemple 4, illustrant en outre la co-localisation de LOX et de NRAGE dans les couches spineuses de l'épiderme de peau humaine normale, avec une juxtaposition de l'expression de LOX et de NRAGE quasi-parfaite. Ainsi, les observations au niveau cellulaire montrent que LOX et NRAGE apparaissent à la périphérie de la cellule (en zone périphérique sousmembranaire), NRAGE apparaissant également au niveau du cytoplasme.

Les inventeurs ont mis ainsi en évidence que les conditions étaient réunies pour que l'interaction directe qu'ils ont mise en évidence *in vitro* puissent s'opérer dans l'épiderme au niveau de la zone de co-localisation de LOX et de NRAGE.

**Exemple 6: mise en évidence de la localisation de LOX et de NRAGE dans la peau de personnes d'âges différents.**

Une étude immuno-histologique utilisant le protocole décrit à l'exemple 4 a été réalisée sur des coupes de peau humaine provenant de 2 donneurs de classes d'ages différents (moins de 20 ans, et supérieur à 60 ans).

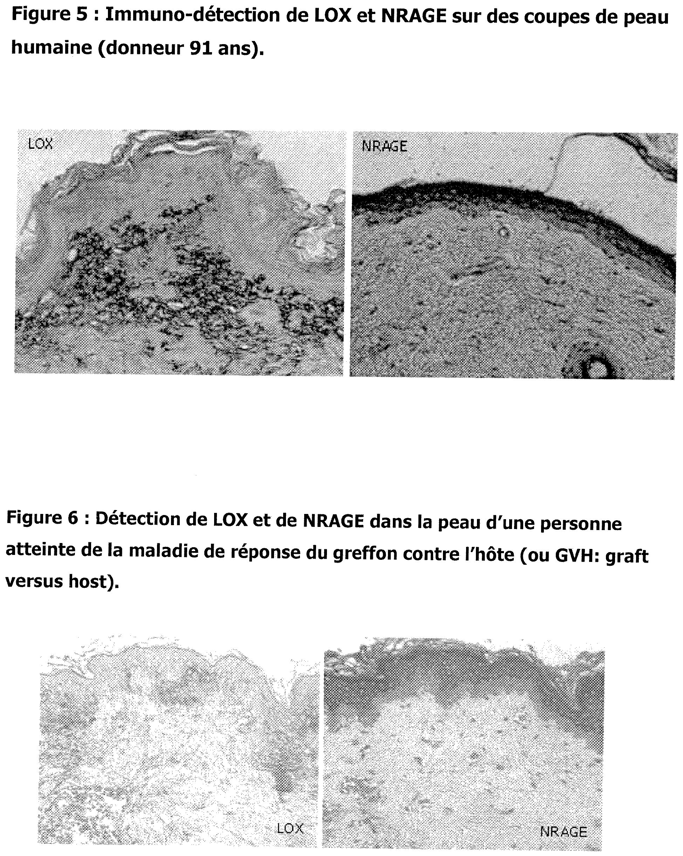

La **Figure 5** présente un marquage effectué sur la peau d'un donneur de 91 ans. Les observations montrent un épiderme hypoprolifératif, très fin (réduit à quelques couches cellulaires), et hyperkératinisé.

Les inventeurs ont mis en évidence la totale absence de LOX (détectable par les techniques utilisées). En revanche, NRAGE est fortement exprimée, localisée au niveau du cytoplasme, et apparaît dès la première couche suprabasale, sans gradient d'expression.

**Exemple comparatif 7: détection de LOX et de NRAGE dans la peau d'une personne atteinte de la maladie de réponse du greffon contre l'hôte (ou GVH: graft versus host).**

Une étude immuno-histologique utilisant le protocole décrit à l'exemple 4 a été réalisée sur des coupes de peaux humaines de patients atteints de la maladie de la réaction de la greffe contre l'hôte (GVH). Cette étude a porté sur 5 donneurs différents.

La **Figure 6** met en évidence une disparition quasi-totale de LOX dans l'épiderme (sans modification de son expression au niveau dermique) et une présence très marquée de NRAGE, localisée au niveau cytoplasmique et apparaissant dès les premières couches suprabasales.

**Exemple comparatif 8: détection de LOX et de NRAGE dans la peau d'une personne présentant un cancer de type basocellulaire et spinocellulaire.**

Une étude immuno-histologique utilisant le protocole décrit à l'exemple 4 a été menée sur des échantillons de peaux de patients présentant un cancer basocellulaire ou spinocellulaire.

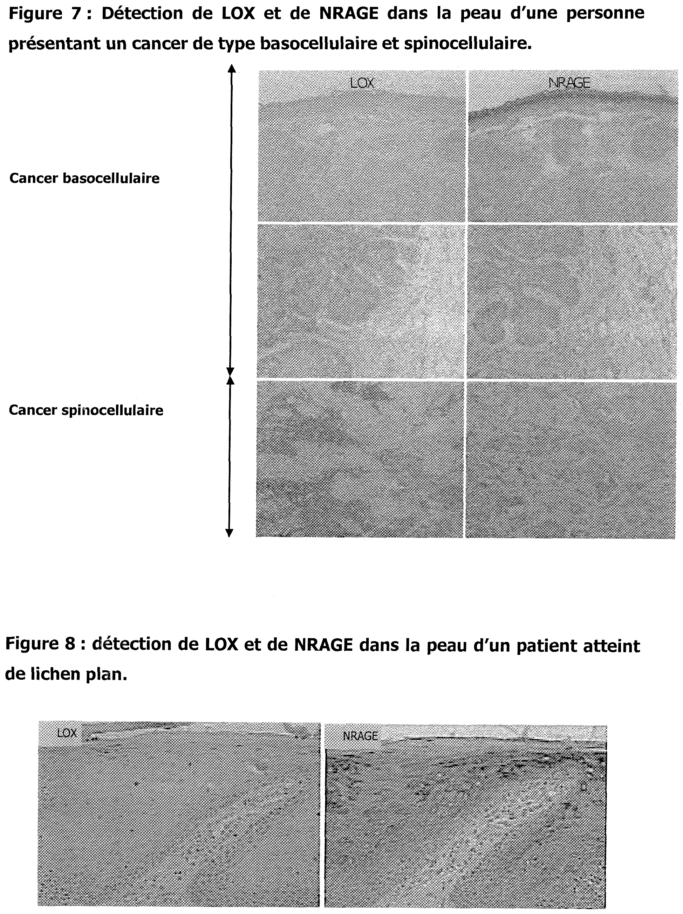

La **Figure 7** met en évidence, dans les deux types de cancer étudiés:

- une diminution progressive de l'expression de LOX et de NRAGE, dans l'épiderme, en périphérie des tumeurs,
- une absence de LOX et de NRAGE au niveau des cellules invasives épidermiques.
- une forte expression de LOX au niveau de la réaction stromale dermique, autour des tumeurs,
- l'absence de NRAGE au niveau de la réaction stromale dermique, autour des tumeurs.

**Exemple comparatif 9: détection de LOX et de NRAGE dans la peau d'un patient atteint de lichen plan.**

Une étude immuno-histologique utilisant le protocole décrit à l'exemple 4 a été réalisée sur des coupes de peau de 3 patients atteints de lichen plan.

La **Figure 8** montre une diminution très forte de l'expression de LOX dans l'épiderme, voire son absence totale. Cette perturbation est corrélée à une irrégularité de l'expression de NRAGE dans l'épiderme.

**Exemple comparatif 10: mise en évidence de la localisation de LOX et de NRAGE dans la peau d'un patient atteint de psoriasis.**

Une étude immuno-histologique utilisant le protocole décrit à l'exemple 4 a été réalisée sur des coupes de peau de 5 patients atteints de psoriasis.

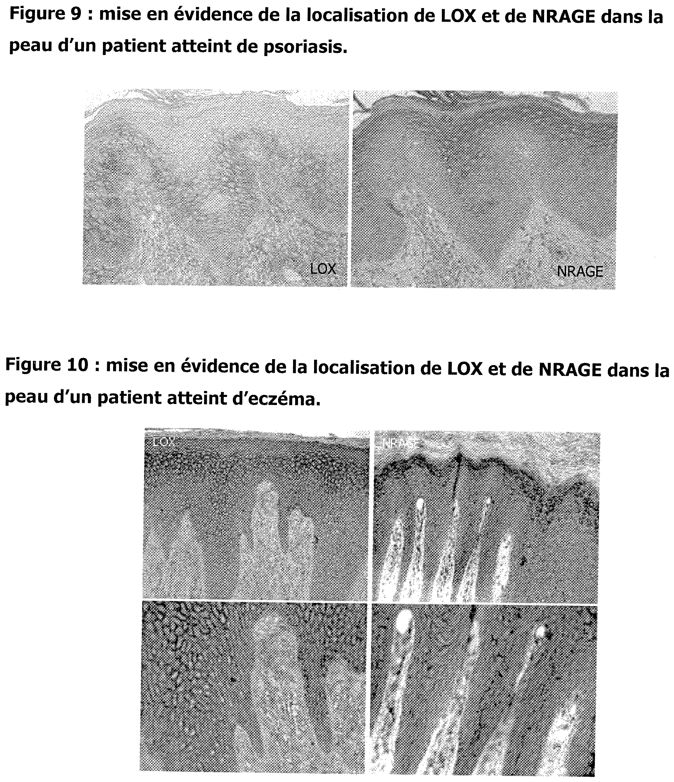

La **Figure 9** est représentative de l'ensemble des coupes observées et montre, au niveau de l'épiderme, une expression très forte de LOX et une présence moyenne de NRAGE, avec un marquage plus ou moins homogène des zones concernées, ne montrant pas de gradient d'expression. A ces caractéristiques d'intensité d'expression variant de la normale, s'ajoutent des anomalies beaucoup plus importantes touchant à la localisation des protéines concernées. Ainsi, dans les peaux psoriatiques, LOX est exprimée essentiellement dans la partie inférieure de l'épiderme et NRAGE uniquement dans sa partie supérieure, l'expression des protéines étant ainsi décalée, sans zone de recouvrement, telle qu'elle est normalement observée dans la peau saine. Au niveau cellulaire, NRAGE est observée au niveau cytoplasmique uniquement et pas en zone périphérique sous-membranaire.

**Exemple comparatif 11: mise en évidence de la localisation de LOX et de NRAGE dans la peau d'un patient atteint d'eczéma.**

Une étude immuno-histologique utilisant le protocole décrit à l'exemple 4 a été réalisée sur des coupes de peau de patients atteints d'eczéma.

La **Figure 10** met en évidence, au niveau épidermique, une très forte expression péricellulaire de LOX. En revanche, NRAGE est peu exprimée et est observée au niveau cytoplasmique uniquement, ce qui traduit une perte de co-localisation au niveau cellulaire.

**Exemple 12: mise en évidence de l'implication de LOX dans l'inhibition de l'apoptose.**

L'étude a été réalisée sur des cultures de kératinocytes humains différenciés en monocouche et à confluence. L'effet de LOX sur l'apoptose a été mis en évidence en inhibant son activité, par ajout de $\beta$-APN (0,02 % p/v - (poids/volume)), en conditions normales ou pro-apoptotiques (choc thermique provoqué par une exposition à une température de 45°C +/-0,5°C pendant 1h30).

La détection et la quantification de la mort cellulaire par apoptose utilisent la technique TUNEL (terminal deoxynucleotidyl transferase mediated dUTP nick end labelling) qui repose sur le marquage des délétions de l'ADN accompagnant l'apoptose.

La première étape consiste à marquer les cassures de l'ADN par TdT (Terminal désoxynucléotidyl transférase) qui catalyse la polymérisation des nucléotides marqués fluorescéine à l'extrémité 3'OH libre de l'ADN. La deuxième étape consiste à détecter la fluorescéine incorporée par un anticorps anti-fluorescéine, conjugué avec la phosphatase alcaline, après incubation avec le substrat de cette dernière.

L'expérimentation est validée à l'aide d'un témoin positif obtenu par fragmentation de l'ADN par une solution de DNase et d'un témoin négatif obtenu en déposant du tampon phosphate.

Les résultats obtenus sont présentés dans le tableau ci-dessous.

**Tableau 1**

|  | Sans choc thermique (% de cellules marquées) | Avec choc thermique (% de cellules marquées) |
|---|---|---|
| Sans β-APN | 6% | 39 % |
| Avec β-APN | 56% | 81% |

Les résultats obtenus démontrent que:

- le choc thermique (45°C +/-0,5°C pendant 1h30) induit une apoptose cellulaire marquée,
- l'inhibition de l'activité enzymatiques de LOX par le β--APN entraîne une augmentation importante de l'apoptose.

Ces résultats mettent en évidence un effet anti-apoptotique de l'activité de LOX.

**Exemple comparatif** 13 **: analyse de l'expression des ARN messagers de LOX et de NRAGE par des kératinocytes en culture, différenciés en milieu calcique, avec et sans mise en contact de principes actifs dont l'activité est à tester (Analyse réalisée, par exemple, par RT-PCR quantitative, Criblage de principes actifs).**

Les actifs ont été testés sur des kératinocytes de prépuces humains normaux de sujets jeunes (pooled normal human epidermal keratinocytes foreskin - Clonetics).

Les kératinocytes sont amplifiés, par exemple, en milieu K-SFM (keratinocytes sérum free medium) supplémenté en antibiotiques jusqu'au troisième passage à 37°C sous 5% de $CO_2$.

Les cellules sont ensemencées en plaques 96 puits, par exemple, à raison de 40 000 cellules par $cm^2$ et cultivées jusqu'à environ 80% de confluence. Les cellules sont ensuite cultivées en milieu hyper-calcique ($CaCl_2$ - 1,7mM à 37°C sous 5% de $CO_2$) afin d'induire une différenciation des cellules.

La matière première utilisée pour la préparation des principes actifs, lorsqu'il s'agit de plantes (de préférence racines, tiges, écorces, fleurs, fruits, graines, germes, gommes, exsudats, feuilles, ou plante entière) ou de protéines, est stérilisée ou non par rayonnement, par exemple beta ou gamma à une dose de préférence de 5kGy puis est réduite en poudre si nécessaire, par exemple par broyage à température ambiante. La poudre est ensuite dispersée à raison de 2 à 5% (poids/poids) de poudre, de préférence 5%, soit dans un solvant polaire, par exemple eau ou butylène glycol, et/ou dans un mélange de solvants polaires, avantageusement un mélange eau/(alcool, glycol ou polyol) (tels que éthanol, glycérol, butylène glycol et autres glycols, xylitol etc..,) en proportions variables et de préférence dans un mélange eau/butylène glycol 75/25 ou 50/50, soit dans un solvant apolaire, comme par exemple un alcane, soit dans un mélange de solvants apolaires, soit dans un mélange de solvants polaires et apolaires. Après agitation pendant au minimum 2 heures sous agitation, par exemple magnétique, l'échantillon est clarifié par décantation ou centrifugation puis filtré de préférence à 0,45μm ou 0,22μm.

La matière première utilisée pour la préparation des principes actifs, lorsqu'il s'agit de molécules caractérisées (par exemple molécules obtenues par synthèse ou hémisynthèse, molécules biologiques obtenues par purification) est diluée dans un solvant, de préférence eau ou diméthylsulfoxide (concentration de préférence comprise entre $10^{-6}$M et de $10^{-2}$M, et préférentiellement de l'ordre de $10^{-4}$M, ou de préférence comprise entre 1% poids/poids et 5% poids/poids, en fonction des molécules). La solution obtenue est ensuite éventuellement filtrée, de préférence, à 0,45μm ou 0,22μm.

Les principes actifs obtenus selon l'une des méthodes décrites ci-dessus sont ensuite testés à une concentration finale de préférence comprise entre 0,01% volume/volume (v/v) et 10 % (v/v) et avantageusement comprise entre 0,1% et 1% (v/v), par exemple à 1% (v/v).

L'incubation en présence des cellules est réalisée avantageusement pendant 24 heures en milieu K-SFM hyper-calcique sans facteurs de croissance. Les cellules sont congelées à sec à -80°C après un rinçage en tampon phosphate pH 7,4.

Extraction des ARNtotaux

Les ARN totaux sont extraits à l'aide du système SV Total RNA Isolation System (Promega, Meylan, France) pour les conditions de plaques 96 puits selon le protocole du fabriquant.

La modification de l'expression des gènes est réalisée par RT-PCR temps réel mesurant l'expression de chaque gène rapportée à l'actine (housekeeping gene) et exprimée en % du témoin négatif non traité.

RT-PCR quantitative en temps réel (Q-RT-PCR)

10μL d'ARNtotaux 5ng/μL sont ajoutés à 40μL de mix PCR (composé de 25μL de SYBR Green Buffer Mix 2X, 0.5μL de mix enzyme, 0.5μM final d'amorce sens et 0.5μM final d'amorce antisens, eau RNase et DNase free qsp 40μL).

La RT-PCR se déroule en différentes étapes dont la retrotranscription à 50°C, 30min, activation de la polymérase 95°C, 15min, réalisation des cycles de PCR (95°C,15s; 60°C, 30s; 72°C,30s) x 50 cycles

Réalisation de la courbe de fusion

    90°C, 1min
    30°C, 1min
    50°C à 95°C, 10s/°C (courbe de fusion)

Le pourcentage de stimulation ou d'inhibition est exprimé par rapport au contrôle non traité (en l'absence de substance à tester).

Gène Actine - Hybridation 60°C

    Sens GTGGGGCGCCCCAGGCACCA (SEQ N° 7)
    Antisens CTCCTTAATGTCACGCACGATTTC (SEQ ID N° 8)

Gène LOX - Hybridation 60°C

    Sens ACGTACGTGCAGAAGATGTCC (SEQ ID N° 3)
    Antisens GGCTGGGTAAGAAATCTGATG (SEQ ID N° 4)

Gène NRAGE - Hybridation 60°C

    Sens TGCACAGACATCAGCAGATGG (SEQ ID N° 5)
    Antisens TTCACGGATGATATCTCTCAGC (SEQ ID N° 6)

Gène Involucrine - Hybridation 60°C

    Sens TGTTCCTCCTCCAGTCAATACCC (SEQ ID N° 9)
    Antisens ATTCCTCATGCTGTTCCCAGTGC (SEQ ID N° 10)

Afin de tenir compte de la population cellulaire présente, tous les résultats ont été rapportés au signal «actine», utilisé comme gène rapporteur (Housekeeping gene). Selon l'expérimentation, le seuil de mesure du C(T) (= Cycle Threshold) a été fixé pour T compris entre 0,05 et 0,01 puis une unité arbitraire de mesure est calculée pour chaque gène selon la formule :

$$\text{Sgène «x»} = 10^7 \times (1/2)^{C(T)\text{gène «x»}}$$

C(T)gène « x » signifiant le nombre de cycles nécessaires pour atteindre le seuil de fluorescence de 0,01-0,05 du gène « x ».

Les valeurs des gènes d'intérêt ont été rapportées au signal « actine » par calcul du rapport:

$$R = S\text{gène « x »} / S\text{actine}.$$

Ces rapports ont été comparés entre les échantillons traités et non traités, « x » étant le gène d'actine, de LOX ou de NRAGE.

Criblage de principes actifs:

Les quantités d'ADNc de chaque essai sont rapportées à la quantité d'ADNc de l'actine puis aux contrôles négatifs (sans actifs). Les résultats sont considérés comme significatifs lorsque l'effet mesuré atteint un facteur d'environ 2. Sur 120 actifs testés, 3 correspondent à ces critères, aux concentrations testées et dans les conditions définies. Ces actifs sont les suivants et font l'objet du tableau ci-dessous :

**Tableau 2**

| Nom | NRAGE Témoin multiplié par: | LOX Témoin multiplié par: |
| --- | --- | --- |
| Ephedra | 2 | 2 |
| Soja | 2,5 | 1 |
| Houblon | 1 | 2 |

De préférence, l'extrait d'éphédra est extrait de la plante entière, notamment par une extraction avec un solvant polaire comme l'eau ou un mélange eau/butylène glycol (par exemple 75/25 ou 50/50), de préférence l'eau.

De préférence, l'extrait de houblon est extrait des cônes, notamment par une extraction avec un solvant polaire comme l'eau ou un mélange eau/butylène glycol (75/25 ou 50/50)), de préférence l'eau.

De préférence, l'extrait de soja est extrait de la graine, notamment par une extraction avec un solvant polaire comme l'eau ou un mélange eau/butylène glycol (75/25 ou 50/50), de préférence l'eau.

Conclusions

A partir de la banque de 120 actifs et dans les conditions considérées:

- 1 actif est capable d'activer significativement le taux de synthèse d'ARNm des gènes codant NRAGE et LOX,
- 1 actif est capable d'activer significativement le taux de synthèse d'ARNm du gène codant NRAGE, sans avoir d'effet sur le gène codant LOX,
- 1 actif est capable d'activer significativement le taux de synthèse d'ARNm du gène codant LOX, sans avoir d'effet sur le gène codant NRAGE.

Cette étude a permis de sélectionner des principes actifs susceptibles de moduler, au moins au niveau des kératinocytes, l'équilibre entre prolifération, différenciation et apoptose.

L'extrait d'éphédra peut être utilisé par exemple dans le traitement de maladies telles que les cancers, de préférence les cancers épithéliaux cutanés (basocellulaires ou spinocellulaires), ou le lichen plan, ou éventuellement pour traiter certaines manifestations cutanées du GVH, ou encore pour réduire les effets du vieillissement sur la peau.

L'extrait de soja peut être utilisé par exemple dans le traitement de maladies telles que les cancers, comme les cancers épithéliaux cutanés (basocellulaires ou spinocellulaires), le GVH, ou le lichen plan, ou pour lutter ou prévenir le vieillissement. L'extrait de soja peut être utilisé notamment pour lutter contre l'hyperprolifération cellulaire.

L'extrait de houblon peut être utilisé par exemple dans le traitement de maladies telles que les cancers, comme les cancers épithéliaux cutanés (basocellulaires ou spinocellulaires), l'eczéma, le psoriasis. L'extrait de houblon peut être utilisé notamment pour lutter contre l'hypoprolifération cellulaire

L'extrait de soja et l'extrait de houblon peuvent être utilisés en association par exemple dans le traitement de maladies telles que les cancers, comme les cancers épithéliaux cutanés (basocellulaires ou spinocellulaires), ou le lichen plan.

L'extrait de soja et l'extrait d'éphédra peuvent être utilisés en association par exemple dans le traitement de maladies telles que les cancers, comme les cancers épithéliaux cutanés (basocellulaires ou spinocellulaires), ou le lichen plan.

L'extrait de houblon et l'extrait d'éphédra peuvent être utilisés en association par exemple dans le traitement de maladies telles que les cancers, comme les cancers épithéliaux cutanés (basocellulaires ou spinocellulaires), ou le lichen plan.

**Exemple comparatif 14 : analyse de la cinétique de l'expression des ARN messagers, par RT-PCR quantitative, de NRAGE et de LOX sur kératinocytes en cours de différenciation calcique. Effet de principes actifs sur la cinétique d'expression.**

Les conditions expérimentales mises en oeuvre pour obtenir des cellules en confluence à 80 % sont identiques à celles décrites à l'exemple comparatif 13. La différenciation s'effectue, en présence de calcium ($CaCl_2$ 1,7 mM) et de principe actif. Une analyse est effectuée après 2, 3 et 4 jours d'incubation par Q-RT-PCR (méthode décrite à l'exemple 13).

Neuf autres substances ont ainsi été testées. L'une d'elles, un extrait de cannelle, permet d'obtenir une inhibition de LOX et de NRAGE, dans les conditions expérimentales considérées.

**Tableau 3**

| Nom | NRAGE Témoin multiplié par | LOX Témoin multiplié par |
|---|---|---|
| Cannelle 2 J | 0,9 | 0,5 |
| 3 J | 0,7 | 0,7 |
| 4 J | 0,2 | 0,5 |
| (J=Jours) | | |

De préférence, l'extrait de cannelle est extrait de l'écorce, notamment par une extraction avec un solvant polaire comme l'eau ou un mélange eau/butylène glycol (75/25 ou 50/50), de préférence l'eau.

Conclusion

L'extrait de cannelle peut être utilisé par exemple dans le traitement de maladies telles que le psoriasis.

L'extrait de cannelle et l'extrait de soja (dont l'effet a été détecté dans l'exemple comparatif précédent) peuvent être utilisés en association par exemple dans le traitement de maladies telles que le traitement de certaines manifestations cutanées du GVH, un eczéma, un psoriasis, ou encore pour réduire les effets du vieillissement sur la peau.

**Exemple 15 : Analyse de l'expression des ARN messagers de LOX par des kératinocytes en culture en l'absence de différenciation calcique, avec et sans mise en contact de principes actifs dont l'activité est à tester (Analyse réalisée, par exemple, par RT-PCR quantitative, Criblage de principes actifs).**

Les actifs ont été testés sur des kératinocytes humains normaux de sujets jeunes obtenus par extraction enzymatique de biopsies humaines collectées après résection chirurgicale et cultivés en monocouches en milieu défini K-SFM (keratinocytes sérum free médium avec suppléments) supplémenté en antibiotiques à 37°C sous 5% de $CO_2$.

Les cellules sont ensemencées au second passage en plaques 24 puits, par exemple, à raison de 30000 cellules par $cm^2$ et cultivées jusqu'à environ 95% de confluence. Les tapis cellulaire sont rincés en tampon phosphate pH 7,4 de préférence avec calcium et magnésium, avant d'être mis en contact avec les principes actifs à tester ou les témoins positifs de référence dilués dans le milieu K-SFM préparés sans suppléments mais avec antibiotiques.

Des principes actifs de différentes origines (végétale, biotechnologique ou molécules de synthèse par exemple) sont testés de 0,1% volume/volume (v/v) à 1% (v/v). les principes actifs d'origine végétale sont testés par exemple à 1%(v/v) et les molécules de synthèse sont testées par exemple à 0,1%(v/v).

En particulier, les principes actifs d'origine végétale sont des extraits qui sont obtenus en faisant macérer les plantes (de préférence racines, rhyzomes, tiges, écorces, fleurs, fruits, graines, germes ou feuilles) à 2-5% (p/p) dans un solvant ou un mélange de solvants, avantageusement un mélange eau/(alcool, glycol ou polyol) (tels que éthanol, glycérol, butylène glycol et autres glycols, xylitol etc..,) de 100/0 à 0/100 (v/v). Les extraits obtenus sont ensuite filtrés ou distillés afin de récupérer la fraction soluble qui est ensuite filtrée à 0,45μm de préférence. Les hydrolysats biotechnologiques sont obtenus par fermentation d'extraits végétaux en présence de microorganismes avantageusement de la famille des Lactobacillus ou Saccharomyces. Ces hydrolysats sont ensuite filtrés de préférence à 0.45μm.

L'incubation est réalisée avantageusement pendant 24 heures à 37°C sous 5% de $CO_2$ en milieu K-SFM sans facteurs de croissance avec antibiotiques. Les témoins négatifs sont soit le milieu de culture seul, soit le milieu de culture contenant 0,1% (v/v) à 1% (v/v) du solvant utilisé lors du procédé d'extraction des extraits testés. Le témoin positif de référence utilisé afin d'induire une différenciation des cellules est une solution de chlorure calcium ($CaCl_2$ - 1,7mM en concentration finale).

Les cellules non traitées (témoin NT) sont congelées à sec à -80°C après le rinçage en tampon phosphate pH 7,4. Après traitement pendant 24h en présence des actifs ou des témoins, les cellules sont congelées à sec à -80°C après le rinçage en tampon phosphate pH 7,4.

Extraction des ARNtotaux

Les ARN totaux sont extraits à l'aide du système SV Total RNA Isolation System (Promega, Meylan, France) pour les conditions de plaques 24 puits selon le protocole du fabriquant.

La modification de l'expression des gènes est réalisée par RT-PCR temps réel mesurant l'expression de chaque gène rapportée à l'actine (housekeeping gene) et exprimée en % du témoin négatif non traité (NT).

RT-PCR quantitative en temps réel (Q-RT-PCR)

10μL d'ARN totaux 5ng/μL sont ajoutés à 40μL de mix PCR (composé de 25μL de SYBR Green Buffer Mix 2X, 0.5μL de mix enzyme, 0.5μM final d'amorce sens et 0.5μM final d'amorce antisens, eau RNase et DNase free qsp 40μL).

La RT-PCR se déroule en différentes étapes dont la retrotranscription à 50°C, 30min, activation de la polymérase 95°C, 15min, réalisation des cycles de PCR (95°C-15s, 60°C-30s, 72°C-30s, 78°C-30s) x 50 cycles.

Réalisation de la courbe de fusion

    90°C, 1min
    30°C, 1min
    50°C à 95°C, 10s/°C (courbe de fusion)

Amorces utilisées :

Gène Actine - Hybridation 60°C

    Sens GTG GGG CGC CCC AGG CAC CA (SEQ ID N° 7)
    Antisens CTC CTT AAT GTC ACG CAC GAT TTC (SEQ ID N° 8)

Gène LOX - Hybridation 60°C

    Sens ACG TAC GTG CAG AAG ATG TCC (SEQ ID N° 3)
    Antisens GGC TGG GTA AGA AAT CTG ATG (SEQ ID N° 4)

Afin de tenir compte de la population cellulaire présente, tous les résultats ont été rapportés au signal «actine», utilisé comme gène de ménage (Housekeeping gene). Selon l'expérimentation, le seuil de mesure du C(T) (= Cycle Threshold) a été fixé pour T compris entre 0,05 et 0,01 puis une unité arbitraire de mesure est calculée pour chaque gène selon la formule :

$$\text{Sgène «LOX»} = 10^7 \times (1/2)\, C(T)\text{gène «LOX»}$$

C(T)gène « LOX » signifiant le nombre de cycles nécessaires pour atteindre le seuil de fluorescence de 0,01-0,05 du gène « LOX ».

Les valeurs des gènes d'intérêt ont été rapportées au signal « actine » par calcul du rapport:

$$R = S\text{gène « LOX »} / S\text{actine}.$$

Ces rapports ont été comparés entre les échantillons traités et non traités.

Criblage de principes actifs:

Les quantités d'ADNc de chaque essai sont rapportées à la quantité d'ADNc de l'actine puis aux contrôles négatifs (NT). Les résultats sont considérés comme significatifs lorsque l'effet mesuré est une modulation d'un facteur d'environ 2 (stimulation) ou 0,5 (inhibition). Sur 60 actifs testés, 30 correspondent à ces critères, dans les conditions définies. Ces actifs sont les suivants et font l'objet du tableau ci-dessous :

**Tableau 1**

| Description | Nom latin | Modulation de LOX vs NT | Partie de la plante Utilisée de préférence |
|---|---|---|---|
| Saponaire blanche | Gypsophila ssp | 0.4 | racine |
| Santal rouge | Pterocarpus santalinus | 0.4 | bois total |
| Bryone | Bryonia dioica | 0.5 | racine |
| Petit Houx | Ruscus aculeatus | 2.0 | racine |
| Citron | Citrus limonia | 2.0 | fruit |
| Mandarine | Citrus reticulata | 2.1 | fruit |
| Ethyl trans 3 hexenoate | hexenoate | 2.3 | - |
| Khella | Amni Visnaga | 2.4 | fruit |
| Acide alginique | | 2.4 | - |
| Carotte | Daucus Carota | 2.4 | racine |
| Methyl 2 methyl butyrat | / | 2.5 | - |
| Badianier de chine | Illicium verum | 2.5 | fruit |
| Cyprès | Cupressus semperviren | 2.6 | fruit |
| Asae Foetida gomme | | 2.6 | - |
| Xylitol | / | 2.8 | - |
| Houblon | Humulus Lupulus | 2.8 | fruit |
| Prunellier | Prunus spinosa | 3.1 | fruit |
| Arbousier | Arbutus unedo | 3.3 | feuille |
| Pyrole | Chimaphila umbellata | 3.4 | plante |
| Asperule odorante | Asperula odorata | 4.7 | plante |
| Armoise | Artemisia vulgaris | 4.7 | racine |

(suite)

| Description | Nom latin | Modulation de LOX vs NT | Partie de la plante Utilisée de préférence |
|---|---|---|---|
| Sureau | Sambucus nigra | 5.0 | fruit |
| Choux chinois | Brassica Brassica campestris var. Pekinen | 5.3 | plante |
| Cannelle | Cinnamomum spp | 5.5 | tige écorce |
| Ephedra | Ephedra sinica | 6.0 | plante |
| Quassia de Surinam | Cassia amara | 6.2 | bois total |
| Cacoyer | Theobroma cacao | 7.4 | coque du fruit |
| Soie | Serica | 7.9 | - |
| Salsepareille rouge | Smilax ornata | 9.0 | racine |
| Groseille | Ribes rubrum | 9.1 | fruit |
| Pyrethre | Anacyclus pyrethrum | 9.8 | racine |
| Fenouil | Foeniculum | 10.2 | plante |

Conclusions

A partir de la banque de 60 actifs et dans les conditions considérées:

- 3 actifs sont capables d'inhiber significativement le taux de synthèse d'ARNm du gène codant LOX,
- 28 actifs dont le Quassia du Surinam, sont capables d'activer significativement le taux de synthèse d'ARNm du gène codant LOX,

Cette étude a permis de sélectionner des principes actifs susceptibles de moduler, au moins au niveau des kératinocytes, l'équilibre entre prolifération, différenciation et apoptose.

**Exemple comparatif 16 : Analyse de l'expression de protéines impliquées dans la régulation de l'homéostasie prolifération/différenciation épidermique dans un modèle de peau reconstruite avec et sans mise en contact de principes actifs dont l'activité est à tester par histologie (exemple l'extrait d'éphédra).**

La mise en évidence de marqueurs de prolifération ou différenciation a été réalisée dans un modèle de peau reconstruite (MIMESKIN®, Engelhard Lyon, France) préparé à partir d'un substrat dermique (collagène/glycosaminoglycannes/chitosane, MIMEDISC®, Engelhard Lyon, France) ensemencé par des fibroblastes humain normaux, à la surface duquel ont été déposés des kératinocytes humains normaux, les cellules étant extraites par traitement enzymatique de biopsies obtenues par résection chirurgicale.

Le modèle de peau reconstruite est en particulier réalisé selon le protocole suivant :

- 0,5 à $1.10^6$ fibroblastes de peau humaine normale sont ensemencés sur un substrat matriciel à base de collagène/glycosaminoglycanne/chitosane, puis cultivés dans un milieu nutritif, par exemple DMEM-Glutamax supplémenté avec 10% de sérum de veau ; acide ascorbique, de préférence à une concentration finale de 1 mM ; EGF (facteur de croissance épidermique), de préférence à une concentration finale de 10 ng/mL ; Normocin, préférence à une concentration finale de 100 $\mu$g/mL, pendant 21 jours.
- 0,5 à $1.10^6$ kératinocytes humains normaux sont ensemencés sur l'équivalent dermique, puis cultivés dans un milieu nutritif, par exemple DMEM-Glutamax/Ham F-12 (ratio 3/1 v/v) supplémenté avec du sérum de veau ; acide ascorbique, de préférence à une concentration finale de 1 mM ; EGF (facteur de croissance épidermique), de préférence à une concentration finale de 10 ng/mL ; hydrocortisone, de préférence à une concentration finale de 0,4 $\mu$g/mL ; umuline, de préférence à une concentration finale de 0,12 UI/mL ; isuprel, de préférence à une concentration finale de 0,4 $\mu$g/mL ; triiodothyronine, de préférence à une concentration finale $2.10^{-9}$ M ; adénine, de préférence à une concentration finale de 24,3 µg/mL ; Normocine, de préférence à une concentration finale de 100 µg/mL. La culture est poursuivie pendant 7 jours en condition immergée. Les cultures sont ensuite placées à l'interface air-liquide pendant 14 jours supplémentaires dans le même milieu que la culture en immersion, excepté le sérum de veau, l'hydrocortisone, l'isuprel, la triiodothyronine et l'umuline.

Le principe actif (extrait d'éphédra) est dilué avantageusement dans les milieux de culture précédemment décrits à 0,5 et 1% et utilisé 3 jours après ensemencement respectif des fibroblastes et des kératinocytes (ie jours 3 à 21 et jours 24 à 42). Le témoin positif est réalisé de préférence par ajout de chlorure de calcium 1.5mM en concentration finale pendant la phase émergée des peaux reconstruites (ie jours 28 à 42) afin de stimuler la différenciation épidermique.

En fin de culture, les échantillons sont congelés, inclus dans une résine thermosensible puis cryo-coupés à 5µm.

Les marquages sur coupes sont réalisés avec les réactifs décrits ci-dessous :

- anticorps primaire anti-transglutaminase humaine
- anticorps primaire anti-cytokeratine 10 humaine
- anticorps secondaires couplés alexa-fluor
- bleu evans
- Dapi

La visualisation des immunomarquages est réalisée en microscopie photonique (Axioskop2plus - Zeiss,germany) et la quantification des immunomarquages transglutaminase a été réalisée par analyse d'image (Lucia - Nikon, France) et les effets du traitement par le principe actif sont évalués (test statistique Holm-sidak, $p < 0.01$).

**Tableau 1:** Quantification de l'intensité de marquage de la transglutaminase.

| INTENSITE | Non Traité | Calcium | Ephedra 0.5% | Ephedra 1% |
|---|---|---|---|---|
| | 0.149 | 0.061 | 0.082 | 0.276 |
| | 0.099 | 0.076 | 0.126 | 0.196 |
| | 0.092 | 0.064 | 0.094 | 0.192 |
| **Moyenne** | **0.113** | **0.067** | **0.100** | **0.221** |
| Ecart type | 0.031 | 0.008 | 0.023 | 0.048 |
| **Variation vs NT** | **100%** | **59%** | **88%** | **196%** |
| Significativité vs NT | - | Non | Non | Oui |
| **Variation vs Ca** | **169%** | **100%** | **149%** | **330%** |
| Significativité vs Ca | Non | - | Non | Oui |

Ces résultats indiquent que le principe actif induit le même type de différenciation épidermique que le témoin positif avec une relation dose-effet. En effet, les peaux traitées présentent un plus grand nombre de couches kératinocytaires exprimant la transglutaminase, en particulier dans la couche dite granuleuse. Par ailleurs, le marquage obtenu est plus intense et plus défini tel qu'illustré dans le grossissement de la partie épidermique. La quantification démontre clairement l'induction de la forme protéique après traitement en particulier à la concentration en principe actif de 1% (induction de 2 fois).

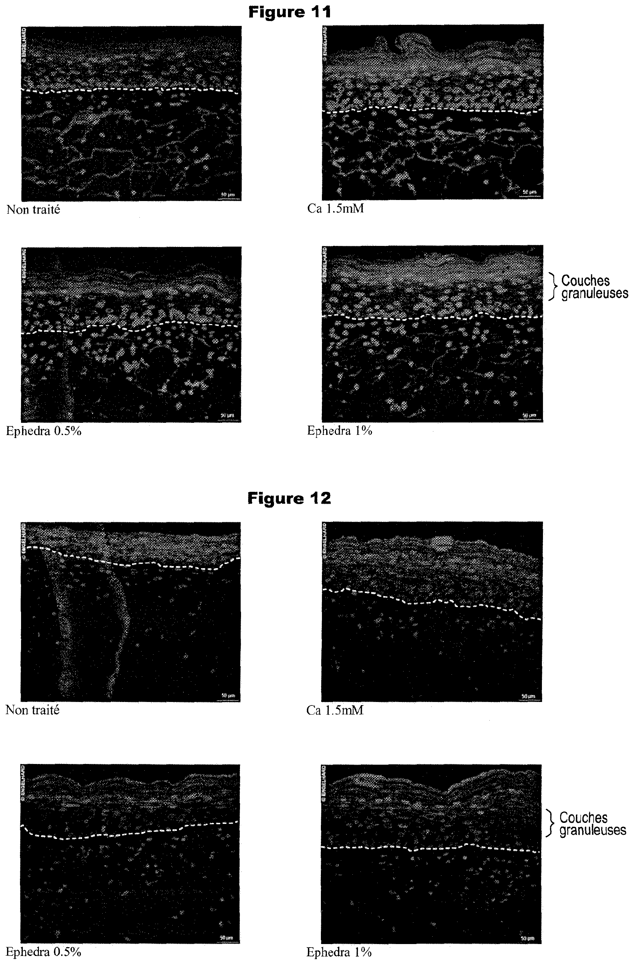

La **Figure 11** présente le marquage globale de la peau reconstruite au bleu Evans (couches épidermique en rouge, cellules en bleu, fibres du substrat dermique en rouge, jonction dermo-épidermique en pointillés).

Ces résultats indiquent que le principe actif induit le même type de différenciation épidermique que le témoin positif avec une relation dose-effet. En effet, les peaux traitées présentent un plus grand nombre de couches kératinocytaires différenciées et en particulier on note une augmentation de l'épaisseur de la couche dite granuleuse. Par ailleurs, un effet intéressant a également été obtenu sur la densité fibroblastique au niveau des dermes traités par l'ephédra.

La **Figure 12** présente la détection immuno-histochimique de la cytokératine 10 sur la peau reconstruite (cellules exprimant fortement le marqueur en rouge, noyaux en bleu, jonction dermo-épidermique en pointillés).

Ces résultats indiquent que le principe actif induit le même type de différenciation épidermique que le témoin positif avec une relation dose-effet. En effet, les peaux traitées présentent un plus grand nombre de couches kératinocytaires exprimant la cyto-keratine 10 et en particulier dans la couche dite granuleuse.

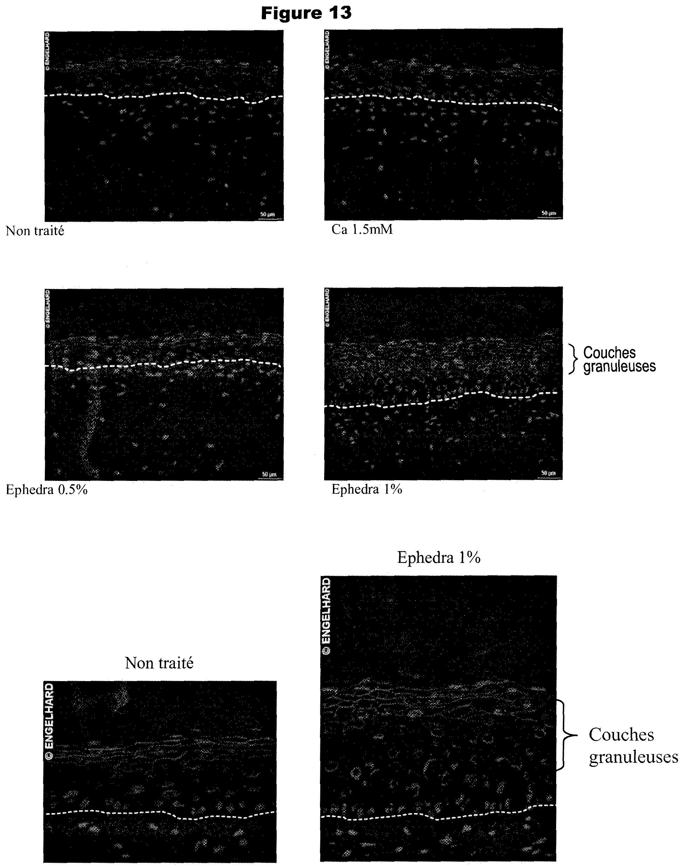

La **Figure 13** présente la détection immuno-histochimique de la transglutaminase sur la peau reconstruite (cellules exprimant fortement le marqueur en rouge, noyaux en bleu, jonction dermo-épidermique en pointillés).

**Exemple 17 : Utilisation des produits de l'invention dans des formulations cosmétiques ou pharmaceutiques de type émulsion huile dans eau**

### Formulation 17a :

| | | |
|---|---|---|
| A | Eau | qsp 100 |
| | Butylene Glycol | 2 |
| | Glycerine | 3 |
| | Sodium Dihydroxycetyl | 2 |
| | Phosphate, | |
| | Isopropyl Hydroxycetyl Ether | |
| | | |
| B | Glycol Stearate SE | 14 |
| | Triisononaoin | 5 |
| | Octyl Cocoate | 6 |
| | | |
| C | Butylene Glycol, | 2 |
| | Methylparaben, | |
| | Ethylparaben, Propylparaben, pH ajusté à 5,5 | |
| | | |
| D | Produits de l'invention | 0,01 - 10 % |

### Formulation 17b :

| | | |
|---|---|---|
| A | Eau | qsp 100 |
| | Butylene Glycol | 2 |
| | Glycerine | 3 |
| | Polyacrylamide, Isoparafin, | 2,8 |
| | Laureth-7 | |
| | | |
| B | Butylene Glycol, | 2 |
| | Methylparaben, | |
| | Ethylparaben, Propylparaben ; | |
| | Phenoxyethanol, | 2 |
| | Methylparaben, | |
| | Propylparaben, Butylparaben, | |
| | Ethylparaben | 0,5 |
| | Butylene Glycol | |
| | | |
| D | Produits de l'invention | 0,01 - 10 % |

### Formulation 17c :

| | | |
|---|---|---|
| A | Carbomer | 0,50 |
| | Propylene Glycol | 3 |
| | Glycerol | 5 |
| | Eau | qsp 100 |
| | | |
| B | Octyl Cocoate | 5 |
| | Bisabolol | 0,30 |
| | Dimethicone | 0,30 |

(suite)

| C | Sodium Hydroxide | 1,60 |
|---|---|---|
| D | Phenoxyethanol, Methylparaben, Propylparaben, Butylparaben, Ethylparaben | 0,50 |
| E | Parfum | 0,30 |
| F | Produits de l'invention | 0,01 - 10 % |

**Exemple 18 de l'invention : Utilisation des produits de l'invention dans une formulation de type eau dans huile**

| A | PEG 30 - dipolyhydroxystearate | 3 |
|---|---|---|
| | Capric Triglycerides | 3 |
| | Cetearyl Octanoate | 4 |
| | Dibutyl Adipate | 3 |
| | Grape Seed Oil | 1,5 |
| | Jojoba Oil | 1,5 |
| | Phenoxyethanol, Methylparaben, Propylparaben, Butylparaben, Ethylparaben | 0,5 |
| B | Glycerine | 3 |
| | Butylene Glycol | 3 |
| | Magnesium Sulfate | 0,5 |
| | EDTA | 0,05 |
| | Eau | qsp 100 |
| C | Cyclomethicone | 1 |
| | Dimethicone | 1 |
| D | Parfum | 0,3 |
| E | Produits de l'invention | 0,01 - 10 % |

**Exemple 19 de l'invention :Utilisation des produits de l'invention dans une formulation de type shampoing ou gel douche**

| A | Xantham Gum | 0,8 |
|---|---|---|
| | Eau | qsp 100 |
| B | Butylene Glycol, Methylparaben, | 0,5 |

| | Ethylparaben, Propylparaben Phenoxyethanol, Methylparaben, Propylparaben, Butylparaben, Ethylparaben | 0,5 |
|---|---|---|
| C | Citric acid | 0,8 |
| D | Sodium Laureth Sulfate | 40,0 |
| E | Produit de l'invention | 0,01 - 10 % |

**Exemple 20 de l'invention :** Utilisation des produits de l'invention dans une formulation de type rouge à lèvres et autres produits anhydres

| A | Mineral Wax | 17,0 |
|---|---|---|
| | Isostearyl Isostearate | 31,5 |
| | Propylene Glycol Dipelargonate | 2,6 |
| | Propylene Glycol Isostearate | 1,7 |
| | PEG 8 Beewax | 3,0 |
| | Hydrogenated Palm Kernel Oil Glycerides, Hydrogenated Palm Glycerides | 3,4 |
| | Lanoline Oil | 3,4 |
| | Sesame Oil | 1,7 |
| | Cetyl Lactate | 1,7 |
| | Mineral Oil, Lanolin Alcohol | 3,0 |
| B | Castor Oil | qsp 100 |
| | Titanium Dioxide | 3,9 |
| | CI 15850 :1 | 0,616 |
| | CI 45410 :1 | 0,256 |
| | CI 19140 :1 | 0,048 |
| | CI 77491 | 2,048 |
| C | Produits de l'invention | 0,01 - 5 % |

**Exemple 21 de l'invention :** Utilisation des produits de l'invention dans une formulation de gels aqueux (contours de l'oeil, amincissants, etc...)

| A | Eau | qsp 100 |
|---|---|---|
| | Carbomer | 0,5 |
| | Butylene Glycol | 15 |
| | Phenoxyethanol, Methylparaben, Propylparaben, Butylparaben, Ethylparaben | 0,5 |

(suite)

| B | Produits de l'invention | 0,01 - 10 % |
|---|---|---|

**Exemple 22de l'invention : Utilisation des produits de l'invention dans une formulation de type émulsion triple**

| | | Emulsion primaire | W1/O |
|---|---|---|---|
| A | | PEG 30 - dipolyhydroxystearate | 4 |
| | | Capric Triglycerides | 7.5 |
| | | Isohexadecane | 15 |
| | | PPG-15 Stéarul ether | 7.5 |
| B | | Eau | 65.3 |
| C | | Phenoxyethanol, Methylparaben, Propylparaben, Butylparaben, Ethylparaben | 0,7 |

| | | Emulsion secondaire W1/O/W2 | |
|---|---|---|---|
| A | | Emulsion primaire | 60 |
| B | | Poloxamer 407 | 2 |
| | | Phenoxyethanol, Methylparaben, Propylparaben,2-bromo-2nitropropane-1,3 diol | 0.3 |
| | | eau | qsp 100 |
| C | | Carbomer | 15 |
| D | | Triethanolamine | PH 6.0-6.5 |

**Exemple comparatif 23: Préparation de formulations pharmaceutiques contenant le produit de l'invention**

**Formulation comparatif 23a : préparation de comprimés**

| A | Excipients | En g par comprimé |
|---|---|---|
| | Lactose | 0,359 |
| | Saccharose | 0,240 |
| B | Produits de l'invention* | 0,001 -0,1 |

*Le produit de l'invention est obtenu, par exemple, selon le procédé d'extraction décrit à l'exemple 13 suivi d'une étape de séchage.

**Formulation comparatif 23b : préparation d'une pommade**

| A | Excipients | |
|---|---|---|
| | Polyéthylène basse densité | 5,5 |

(suite)

| | | |
|---|---|---|
| | Paraffine liquide | qsp 100 |
| B | Produits de l'invention* | 0,001 -0,1 |

*Le produit de l'invention est obtenu, par exemple, selon le procédé d'extraction décrit à l'exemple 13 suivi d'une étape de séchage.

### Formulation comparatif 23c : préparation d'une formule injectable

| | | |
|---|---|---|
| A | Excipient | |
| | Solution isotonique salée | 5 ml |
| B | Produits de l'invention* | 0,001 -0,1 g |

*Le produit de l'invention est obtenu, par exemple, selon le procédé d'extraction décrit à l'exemple 13 suivi d'une étape de séchage.

### Exemple 24: Evaluation de l'acceptation cosmétique d'une préparation contenant le sujet de l'invention

Les essais de toxicologie ont été réalisés sur le composé obtenu selon l'exemple 2 incorporé à 10% dans un gel de xanthane à 0.5%, par une évaluation oculaire chez le lapin, par l'étude de l'absence de toxicité anormale par administration orale unique chez le rat et par l'étude du pouvoir sensibilisant sur le cobaye.

### Evaluation de l'irritation primaire cutanée chez le lapin :

Les préparations décrites ci-dessus sont appliquées sans dilution à la dose de 0,5 ml sur la peau de 3 lapins selon la méthode préconisée par la directive OCDE concernant l'étude de « l'effet irritant/corrosif aigu sur la peau ».
Les produits sont classés selon les critères définis par l'arrêté du 1/2/1982 publié au JORF du 21/02/82.
Les résultats de ces essais, ont permis de conclure que les produits de l'invention étaient classés **non irritants** pour la peau.

### Evaluation de l'irritation oculaire chez le lapin :

Les préparations décrites ci-dessus ont été instillées pure en une seule fois, à raison de 0,1ml, dans l'oeil de 3 lapins selon la méthode préconisée par la directive de l'OCDE n °405 du 24 février 1987 concernant l'étude de « l'effet irritant/corrosif aigu sur les yeux ».
Les résultats de ce test permettent de conclure que les préparations peuvent être considérées comme **non irritantes** pour les yeux, au sens de la directive 91/326 CEE utilisée pure ou sans dilution.

### Essai sur l'absence de toxicité anormale par administration orale unique chez le rat :

Les préparations décrites ont été administrées en une fois par voie orale à la dose de 5g/Kg de poids corporel, à 5 rats mâles et 5 rats femelles selon un protocole inspiré de la directive de l'OCDE n°401 du 24 février 1987 et adapté aux produits cosmétiques.
Les DL0 et DL50 sont trouvées supérieures à 5000 mg/Kg. Les préparation s testées ne sont donc pas classées parmi les préparations dangereuses par ingestion.

### Evaluation du potentiel de sensibilisation cutanée chez le cobaye :

Les préparations décrites sont soumises au test de maximisation décrit par Magnusson et Kligmann, protocole en accord avec la ligne directrice n°406 de l'OCDE.
Les préparations sont classées comme non sensibilisantes par contact avec la peau.

**Revendications**

1. Utilisation d'une substance stimulant l'expression de la protéine LOX ayant la séquence ID N°1 pour le traitement ou la prévention du vieillissement de la peau, ladite substance étant un extrait de bois total de Quassia de Surinam (Cassia amara), ladite substance étant préparée sous forme d'une composition cosmétique.

2. Utilisation selon la revendication 1, **caractérisée en ce que** la composition cosmétique comprend de 0,01% et 10% (v/v) de ladite substance.

3. Utilisation selon la revendication 1, **caractérisée en ce que** la composition cosmétique comprend de 0,1% et 1% (v/v) de ladite substance.

4. Utilisation, selon l'une quelconque des revendications 1 à 3, **caractérisée en ce qu'**une plante est réduite en poudre et la poudre est dispersée à raison de 2 à 5% (poids/poids) de poudre dans un solvant polaire, avantageusement un mélange eau/(alcool, glycol ou polyol), et de préférence dans un mélange eau/butylène glycol 75/25 ou 50/50, pour préparer ladite substance.

5. Utilisation, selon l'une quelconque des revendications 1 à 4, **caractérisée en ce que** ledit extrait de Quassia de Surinam (Cassia amara) est obtenu en faisant macérer la plante à 2-5 % (p/p) dans un solvant ou un mélange de solvants, l'extrait obtenu étant ensuite filtré ou distillé afin de récupérer la fraction soluble ensuite filtrée à 0,45μm de préférence, ledit extrait étant capable d'activer significativement le taux de synthèse d'ARNm du gène codant LOX par des kératinocytes en culture en l'absence de différenciation calcique.

6. Utilisation, selon l'une quelconque des revendications 1 à 5, **caractérisée en ce que** la composition comprend au moins un excipient choisi parmi le groupe consistant en les conservateurs, les émollients, les émulsifiants, les tensioactifs, les hydratants, les épaississants, les conditionneurs, les agents matifiants, les stabilisants, les antioxydants, les agents de texture, les agents de brillance, les agents filmogènes, les solubilisants, les pigments, les colorants, les parfums et les filtres solaires.

**Patentansprüche**

1. Verwendung einer Substanz, die die Expression des Proteins LOX stimuliert, die die Sequenz ID Nr.1 zur Behandlung oder Vorbeugung der Hautalterung aufweist, wobei die Substanz ein totaler Holzextrakt von Quassia de Surinam (Cassia amara) ist, wobei die Substanz in Form eine kosmetischen Zusammensetzung hergestellt ist.

2. Verwendung gemäß Anspruch 1, **dadurch gekennzeichnet, dass** die kosmetische Zusammensetzung 0,01 % und 10 % (v/v) der Substanz umfasst.

3. Verwendung gemäß Anspruch 1, **dadurch gekennzeichnet, dass** die kosmetische Zusammensetzung 0,1 % und 1 % (v/v) der Substanz umfasst.

4. Verwendung gemäß einem der Ansprüche 1 bis 3, **dadurch gekennzeichnet, dass** eine Pflanze zu Pulver reduziert wird, und das Pulver im Verhältnis von 2 bis 5 % (Gewicht/Gewicht) Pulver in einem polaren Lösungsmittel, vorteilhafterweise einem Gemisch Wasser/(Alkohol, Glycol oder Polyol), und vorzugsweise in einem Gemisch Wasser/Butylen Glycol 75/25 oder 50/50, dispergiert wird, um die Substanz herzustellen.

5. Verwendung nach einem der Ansprüche 1 bis 4, **dadurch gekennzeichnet, dass** der Extrakt von Quassia de Surinam (Cassis amara) erhalten wird, wobei die Pflanze zu 2-5 % (Gew./Gew.) in ein Lösungsmittel oder ein Lösungsmittelgemisch eingelegt wird, wobei der erhaltene Extrakt sodann gefiltert oder destilliert wird, um die lösliche, sodann auf vorzugsweise 0,45 μm gefilterte Fraktion zu gewinnen, wobei der Extrakt geeignet ist, die Syntheserate von mRNA des für LOX codierenden Gens durch Keratinocyten in Kultur bei Fehlen einer Kalziumdifferenzierung signifikant zu aktivieren.

6. Verwendung gemäß einem der Ansprüche 1 bis 5, **dadurch gekennzeichnet, dass** die Zusammensetzung mindestens einen Träger umfasst, der ausgewählt ist in der Gruppe, bestehend aus den Konservierungsmitteln, Weichmachern, Emulgatoren, Tensiden, Feuchtigkeitsspendern, Verdickungsmitteln, Konditionierern, Mattierungsmitteln, Stabilisatoren, Antioxidantien, Texturierungsmitteln, Glanzbildnern, Filmbildnern, Solubilisierungsmitteln, Pigmenten, Farbstoffen, Parfums und den Sonnenfiltern.

**Claims**

1. Use of a substance stimulating the expression of LOX protein having sequence ID no. 1 for the treatment or prevention of skin ageing, said substance being a total wood extract of Bitter ash (Cassia amara), said substance being prepared in the form of a cosmetic composition.

2. Use according to claim 1, **characterized in that** the cosmetic composition comprises from 0.01% to 10% (v/v) of said substance.

3. Use according to claim 1, **characterized in that** the cosmetic composition comprises from 0.1% to 1% (v/v) of said substance.

4. Use according to any of claims 1 to 3, **characterized in that** a plant is reduced to powder and the powder is dispersed at a rate of 2 to 5% (weight/weight) in a polar solvent, advantageously a mixture of water/(alcohol, glycol or polyol), and preferably in a 75/25 or 50/50 water/butylene glycol mixture, for preparing said substance.

5. Use according to any of claims 1 to 4, **characterized in that** said extract of Bitter ash (Cassia amara), is obtained by macerating the plant at 2-5% (w/w) in a solvent or a mixture of solvents, the obtained extract being then filtered or distilled in order to recover the soluble fraction which is then filtered preferably at 0.45 $\mu$m, said extract being capable of activating the synthesis rate of mRNA of the LOX coding gene by keratinocytes in culture in the absence of calcium differentiation

6. Use according to any of claims 1 to 5, **characterized in that** the composition comprises at least one excipient selected from the group consisting of preservatives, emollients, emulsifiers, surfactants, moisturizers, thickeners, conditioners, matting agents, stabilizers, antioxidants, texturizing agents, brightening agents, film-forming agents, solubilizers, pigments, colorants, perfumes and sun filters

## Figure 1 : Séquence et schéma de la protéine NRAGE (Neurotrophin-Receptor-interactig MAGE homolog ou MAGE D1)

```
MAQKMDCGAGLLGFQAEASVEDSALLMQTLMEAIQISEAPPTNQATAAASPQSSQPPTANEM
ADIQVSAAAARPKSAFKVQNATTKGPNGVYDFSQAHNAKDVPNTQPKAAFKSQNATSKGPNA
AYDFSQAATTGELAANKSEMAFKAQNATTKVGPNATYNFSQSLNANDLANSRPKTPFKAWND
TTKAPTADTQTQNVNQAKMATSQADIETDPGISEPDGATAQTSADGSQAQNLESRTIIRGKR
TRKINNLNVEENSSGDQRRAPLAAGTWRSAPVPVTTQNPPGAPPNVLWQTPLAWQNPSGW
QNQTARQTPPARQSPPARQTPPAWQNPVAWQNPVIWPNPVIWQNPVIWPNPIVWPGPVVW
PNPLAWQNPPGWQTPPGWQTPPGWQGPPDWQGPPDWPLPPDWPLPPDWPLPTDWPLPP
DWIPADWPIPPDWQNLRPSPNLRPSPNSRASQNPGAAQPRDVALLQERANKLVKYLMLKDYT
KVPIKRSEMLRDIIREYTDVYPEIIERACFVLEKKFGIQLKEIDKEEHLYILISTPESLAGILGTTKD
TPKLGLLLVILGVIFMNGNRASEAVLWEALRKMGLRPGVRHPLLGDLRKLLTYEFVKQKYLDYR
RVPNSNPPEYEFLWGLRSYHETSKMKVLRFIAEVQKRDPRDWTAQFMEAADEALDALDAAAA
EAEARAEARTRMGIGDEAVSGPWSWDDIEFELLTWDEEGDFGDPWSRIPFTFWARYHQNAR
SRFPQTFAGPIIGPGGTASANFAANFGAIGFFWVE
```

Nt⊏══▭MHD-2▭──⊏IRD⊨─⊏═══MHD-1═══▭▭══⊏▭═══⊐Ct

| MHD-1 | MAGE Homolgy Domain |
| IRD | Interspersed Repeat Domain |
| | Zones riches en résidus lysines |

## Figure 2 : Interaction en double hybride mammifère

Rapport d'activité luciférase par rapport au contrôle pBind

pAct    NRAGE

**Figure 3 : Mise en évidence de la présence de LOX et de NRAGE dans la peau reconstruite (Objectif 25).**

LOX          NRAGE

**Figure 4 : Co-locolisation en microscopie confocale de LOX et NRAGE sur coupes de peau humaine.**

**Figure 5 : Immuno-détection de LOX et NRAGE sur des coupes de peau humaine (donneur 91 ans).**

**Figure 6 : Détection de LOX et de NRAGE dans la peau d'une personne atteinte de la maladie de réponse du greffon contre l'hôte (ou GVH: graft versus host).**

**Figure 7 : Détection de LOX et de NRAGE dans la peau d'une personne présentant un cancer de type basocellulaire et spinocellulaire.**

**Cancer basocellulaire**

**Cancer spinocellulaire**

**Figure 8 : détection de LOX et de NRAGE dans la peau d'un patient atteint de lichen plan.**

**Figure 9 : mise en évidence de la localisation de LOX et de NRAGE dans la peau d'un patient atteint de psoriasis.**

**Figure 10 : mise en évidence de la localisation de LOX et de NRAGE dans la peau d'un patient atteint d'eczéma.**

# Figure 11

Non traité

Ca 1.5mM

Ephedra 0.5%

Ephedra 1% } Couches granuleuses

# Figure 12

Non traité

Ca 1.5mM

Ephedra 0.5%

Ephedra 1% } Couches granuleuses

# Figure 13

Non traité

Ca 1.5mM

Ephedra 0.5%

Ephedra 1%

} Couches granuleuses

Ephedra 1%

Non traité

} Couches granuleuses

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2828206

**Littérature non-brevet citée dans la description**

- **CSIZAR.** Lysyl oxydases : A novel multifunctional amine oxydase familty. *Nucleic Acid research and Molecular Biology,* 2001, vol. 70, 2-28
- **JEAY et al.** Lysyl oxydase inhibits Ras-mediated transformation by preventing activation of NF-KB. *Mol. Cell. Biol.,* 2003, vol. 23, 2251-2263
- **NOBLESSE.** Lysyl oxydase like and lysyl oxydase are present in the dermis and epidermis of a skin equivalent and in human skin and are associated to elastic fibers. *J. Invest. Dermatol.,* 2004, vol. 122, 621-630
- **PEYROL et al.** Lysyl oxidase gene expression in the stromal reaction to in situ and invasive ductal breast carcinoma. *Am J Pathol,* Février 1997, vol. 150 (2), 497-507
- **LI et al.** Localization and activity of lysyl oxidase within nuclei of fibrogenic cells. *Proc Natl Acad Sci USA.,* 25 Novembre 1997, vol. 94 (24), 12817-22
- **CONTENTE et al.** Expression of gene rrg is associated with reversion of NIH 3T3 transformed by LTR-c-H-ras. *Science,* 1990, vol. 249, 796-798
- **CSISZAR et al.** Somatic mutations of the lysyl oxidase gene on chromosome 5q23.1 in colorectal tumors. *Int. J. Cancer,* 2002, vol. 97, 636-642
- **PALAMAKUMBURA et al.** Autocrine growth factor regulation of lysyl oxidase expression in transformed fibroblasts. *J Biol Chem.,* 15 Août 2003, vol. 278 (33), 30781-7
- **JEAY et al.** Lysyl oxidase inhibits Ras-mediated transformation by preventing activation of NF-B. *Mol. Cell. Biol.,* 2003, vol. 23, 2251-2263
- **NOBLESSE et al.** Lysyl oxydase-like and lysyl oxydase are present in the dermis and epidermis of a skin equivalent and in human skin and are associated to elastic fibers. *J. Invest. Dermatol.,* 2004, vol. 122, 621-630
- **SOMMER et al.** Transient expression of lysyl oxidase by liver myofibroblasts in murine schistosomiasis. *Laboratory Investigation,* 1993, vol. 69, 460-470